# EUROPEAN PATENT SPECIFICATION

(11) **EP 4 182 467 B1**
(45) Date of publication and mention of the grant of the patent: **11.02.2026**
(21) Application number: 21842894.4
(22) Date of filing: 14.07.2021
(51) Int. Cl.: C12N 15/85

(54) **RECOMBINANT ADENO-ASSOCIATED VIRAL VECTORS FOR MULTIPARTITE GENE DELIVERY**
REKOMBINANTE ADENO-ASSOZIIERTE VIRALE VEKTOREN ZUR MULTILATERALEN GENVERABREICHUNG
VECTEURS VIRAUX ADÉNO-ASSOCIÉS RECOMBINÉS POUR LA DÉLIVRANCE DE GÈNES MULTIPLES

(30) Priority: 14.07.2020 US 202063051721 P; 26.04.2021 US 202163179612 P
(43) Date of publication of application: 24.05.2023
(73) Proprietor: Abeona Therapeutics Inc., New York, New York 10019 (US)
(72) Inventor: PADEGIMAS, Linas, New York, New York 10019 (US); BARRETT, Brianna, New York, New York 10019 (US)
(74) Representative: Cooley (UK) LLP
(86) International application number: PCT/US2021/041527
(87) International publication number: WO 2022/015788

(56) References cited:
- WO-A1-2020/127831
- WO-A1-2020/127831
- US-A1- 2002 102 714
- US-A1- 2014 256 802
- US-A1- 2016 076 054
- CHENG YUHSIANG: "Development of a Novel Dual AAV System for Therapeutic Delivery of BDNF", MASTER'S THESIS, UNIVERSITY OF CALIFORNIA SAN DIEGO, 1 January 2018 (2018-01-01), XP055898994, Retrieved from the Internet <URL:https://escholarship.org/uc/item/3v16w70m> [retrieved on 20220308]
- CHENG YUHSIANG: "Development of a Novel Dual AAV System for Therapeutic Delivery of BDNF", MASTER'S THESIS, UNIVERSITY OF CALIFORNIA SAN DIEGO, 1 January 2018 (2018-01-01), XP055898994
- BRIANNA BARRETT; SCOTT KERNS; BRIAN KEVANY; LINAS PADEGIMAS: "Dual AAV vector strategy for expression of large genes targeted for Stargardt disease gene therapy development", INVESTIGATIVE OPHTHALMOLOGY & VISUAL SCIENCE, vol. 62, 31 May 2021 (2021-05-31), US , pages 1484, XP009542894, ISSN: 0146-0404

## Description

### BACKGROUND

Adeno-associated viral (AAV) vectors are promising delivery vectors for gene therapy. However, their therapeutic efficacy is undermined by limitations regarding delivery of genes above a certain size. For example, Stargardt disease is an inherited retinal disease that causes juvenile macular degeneration. Autosomal recessive Stargardt disease is caused by mutations in the ABCA4 gene. The ORF of the human ABCA4 gene is about 6.8 kb in length, which exceeds the packaging capacity of about 4.7 kb typical of AAV vectors. WO2020/127831 describes a novel acceptor splice region, and uses and applications thereof, such as the reconstitution of split AAV vectors, AAV vector systems comprising 2 AAV vectors and/or trap vectors. Cheng Yuhsiang (01-01-2018) discloses a novel regulatable dual AAV vector system for therapeutic delivery of BDNF that can be turned off by oral administration of tamoxifen to adult mice.

Therefore, there is an urgent need for AAV viral vector approaches for providing large genes.

### SUMMARY

The invention is defined by the claims. Any other instances as set forth herein not falling within the scope of the claims are for information only. Any reference to methods of treatment is to be interpreted as a reference to the claimed invention for use in such a method of treatment.

The present disclosure relates generally to the field of gene therapy and in particular, to recombinant adeno-associated viral (AAV) vector particles (also known as AAV viral vectors) their use to deliver transgenes in a multipartite (e.g., bipartite) approach to treat or prevent a disease or disorder.

**In** one aspect of the invention, the present disclosure provides AAV vector genomes comprising, in 5' to 3' orientation:
(a) a 5' AAV inverted terminal repeat;
(b) a promoter;
(c) a 5' portion of a transgene;
(d) a splice donor (SD) site;
(e) a recombination site;
(f) a polynucleotide encoding a recombinase;
(g) a poly-A site; and
(h) a 3' AAV inverted terminal repeat,
wherein expression of the recombinase is operably linked to the promoter.

In embodiments, the recombinase is a Cre recombinase. In embodiments, the recombinase comprises a nuclear localization sequence (NLS). In embodiments, the recombination site comprises a LoxP71 sequence. In embodiments, the AAV vector genome comprises a polynucleotide encoding an internal cleavage polypeptide located between the recombination site and the polynucleotide encoding the recombinase, and wherein the 5' portion of the transgene, the polynucleotide encoding the internal cleavage polypeptide, and the polynucleotide encoding the recombinase are in the same reading frame. In embodiments, the internal cleavage polypeptide is a self-cleaving peptide selected from the group consisting of T2A, P2A, E2A and F2A. In embodiments, the AAV vector genome comprises an internal ribosome entry site (IRES) located between the recombination site and the polynucleotide encoding the recombinase, and wherein the IRES is operably linked to the polynucleotide encoding the recombinase.

In embodiments, the transgene encodes a polypeptide. In embodiments, the polypeptide is an ABCA4 protein.

In embodiments, the promoter is a Rous sarcoma virus (RSV) LTR promoter (optionally with the RSV enhancer), a cytomegalovirus (CMV) promoter, an SV40 promoter, a dihydrofolate reductase promoter, a beta-actin promoter, a phosphoglycerol kinase (PGK) promoter, a U6 promoter, an H1 promoter, a CAG promoter, a hybrid chicken beta-actin promoter, an MeCP2 promoter, an EF1 promoter, a ubiquitous chicken β-actin hybrid (CBh) promoter, a U1a promoter, a U1b promoter, an MeCP2 promoter, an MeP418 promoter, an MeP426 promoter, a minimal MeCP2 promoter, a VMD2 promoter, an mRho promoter, a EFla promoter, a Ubc promoter, a human β-actin promoter, a TRE promoter, an Ac5 promoter, a Polyhedrin promoter, a CaMKIIa promoter, Gal1 promoter, a TEF1 promoter, a GDS promoter, an ADH1 promoter, a Ubi promoter, or an α-1-antitrypsin (hAAT) promoter. In embodiments, the promoter is a human rhodopsin kinase (RK) promoter, a human interphotoreceptor binding protein promoter (IRBP), a human red/green opsin promoter (pR2.1), a human blue opsin promoter (HB), a mouse opsin promoter (mOP), a mouse short wavelength opsin promoter (mBP), or a human rod cGMP phosphodiesterase β-subunit promoter (βPDE).

In one aspect provided for illustrative purposes, which is not claimed, the present disclosure provides AAV vector genomes comprising, in 5' to 3' orientation:
(a) a 5' AAV inverted terminal repeat;
(b) a recombination site;
(c) a splice acceptor (SA) site;
(d) a 3' portion of a transgene;
(e) a poly-A site; and
(f) a 3' AAV inverted terminal repeat.

In one aspect, the present disclosure provides AAV vectors comprising an AAV vector genome of the disclosure. In one aspect, the present disclosure provides polynucleotides comprising an AAV vector genome of the disclosure.

In one aspect, the present disclosure provides AAV viral particles comprising (i) an AAV capsid, wherein the AAV capsid comprises an AAV capsid protein; and (ii) a AAV vector genome of the disclosure. In embodiments, the AAV capsid protein is at least 70%, 80%, 90%, 99% or 100% identical to a sequence selected from SEQ ID NOs: 1-3, 67, 71, 196, 205 and 206.

In one aspect, the present disclosure provides pharmaceutical compositions comprising the AAV vector genome of the disclosure or the AAV viral particle of the disclosure. In embodiments, the pharmaceutical composition comprises:
(i) a first AAV viral particle comprising the AAV vector genome of the disclosure; and
(ii) a second AAV viral particle comprising a second AAV vector genome comprising, in 5' to 3' orientation:
   (a) a 5' AAV inverted terminal repeat;
   (b) a recombination site;
   (c) a splice acceptor (SA) site;
   (d) a 3' portion of the transgene;
   (e) a poly-A site; and
   (f) a 3' AAV inverted terminal repeat;
wherein the recombination site in the AAV vector genome of the first AAV viral particle and the recombination site in the second AAV vector genome of the second AAV viral particle prevent backwards recombination.

In one aspect, the disclosure provides methods of treating a subject having a disease or disorder caused by a gene defect, comprising:
(1) administering to the subject a first AAV viral particle comprising:
   (i) an AAV capsid, wherein the AAV capsid comprises a first AAV capsid protein; and
   (ii) the AAV vector genome of the disclosure;
(2) administering to the subject a second AAV viral particle, comprising:
   (i) an AAV capsid, wherein the AAV capsid comprises a second AAV capsid protein; and
   (ii) a second AAV vector genome, wherein the second AAV vector genome comprises, in 5' to 3' orientation:
      (a) a 5' AAV inverted terminal repeat;
      (b) a recombination site;
      (c) a splice acceptor (SA) site;
      (d) a 3' portion of the transgene;
      (e) a poly-A site; and
      (f) a 3' AAV inverted terminal repeat.
In embodiments, administration of the first AAV viral particle and the second AAV viral particle leads to recombination of the first AAV vector genome and the second AAV vector genome via the recombination site in the AAV vector genome of the first AAV viral particle and the recombination site in the second AAV vector genome of the second AAV viral particle. In embodiments, administration of the first AAV viral particle and the second AAV viral particle leads to expression of the polypeptide. In embodiments, the first AAV viral particle and the second AAV viral particle are co-administered or sequentially administered. In embodiments, the first AAV viral particle and the second AAV viral particle are co-administered. In embodiments, the viral particles are administered by subretinal injection. In embodiments, the gene defect is an ABCA4 gene defect. In embodiments, the ABCA4 gene defect results in one or more conditions selected from the group consisting of reduced expression of ABCA4 protein, eliminated expression of ABCA4 protein, expression of a mutated ABCA4 protein, and reduced function of ABCA4 protein.

In one aspect, the disclosure provides methods of expressing a polypeptide in a cell, comprising:
(1) transducing the cell with a first AAV viral particle comprising the AAV vector genome of the disclosure, wherein the transgene encodes the polypeptide; and
(2) transducing the cell with a second AAV viral particle comprising a second AAV vector genome, wherein the second AAV vector genome comprises, in 5' to 3' orientation:
   (a) a 5' AAV inverted terminal repeat;
   (b) a recombination site;
   (c) a splice acceptor (SA) site;
   (d) a 3' portion of the transgene;
   (e) a poly-A site; and
   (f) a 3' AAV inverted terminal repeat.
In embodiments, no stable expression of the recombinase is detected in the transduced cell. In embodiments, the polypeptide is an ABCA4 protein.

In one aspect, the disclosure provides transduced cells comprising:
(i) a defective genomic copy of a gene;
(ii) a first recombinant nucleic acid comprising, in 5' to 3' orientation:
   (a) a 5' AAV inverted terminal repeat;
   (b) a promoter;
   (c) a 5' portion of a transgene;
   (d) a splice donor (SD) site;
   (e) a recombination site;
   (f) a splice acceptor (SA) site;
   (g) a 3' portion of the transgene;
   (h) a poly-A site; and
   (i) a 3' AAV inverted terminal repeat;
   wherein the transgene has a length of 4.6-7.7 kb; and
(iii) a second recombinant nucleic acid comprising, in 5' to 3' orientation: a 5' AAV inverted terminal repeat, a polynucleotide encoding a recombinase, and a 3' AAV inverted terminal repeat; wherein the second recombinant nucleic acid lacks a promoter.
In embodiments, no stable expression of the recombinase is detected in the transduced cell. In embodiments, the recombinase is a Cre recombinase. In embodiments, the transgene encodes an ABCA4 gene. In embodiments, the cell is an *ex vivo* cell.

### BRIEF DESCRIPTION OF THE DRAWINGS

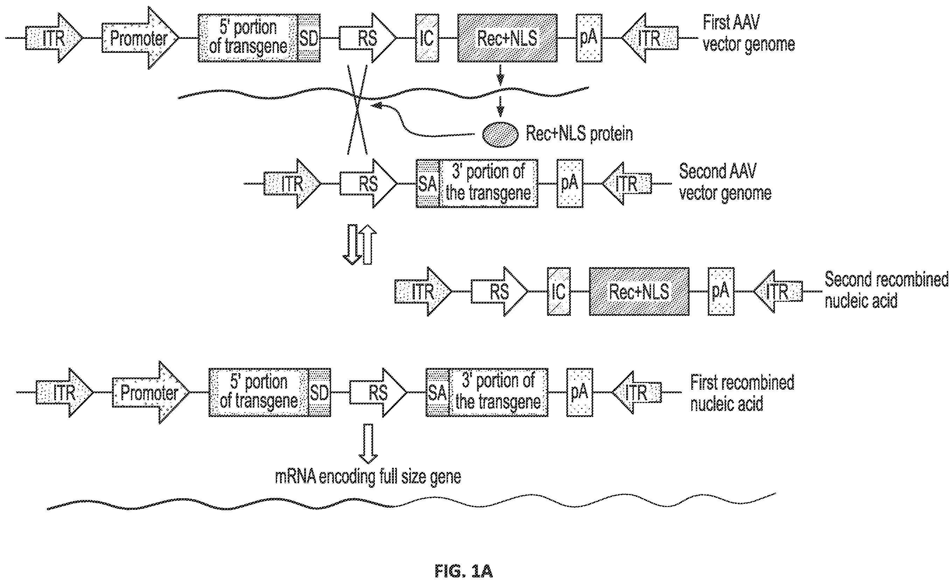

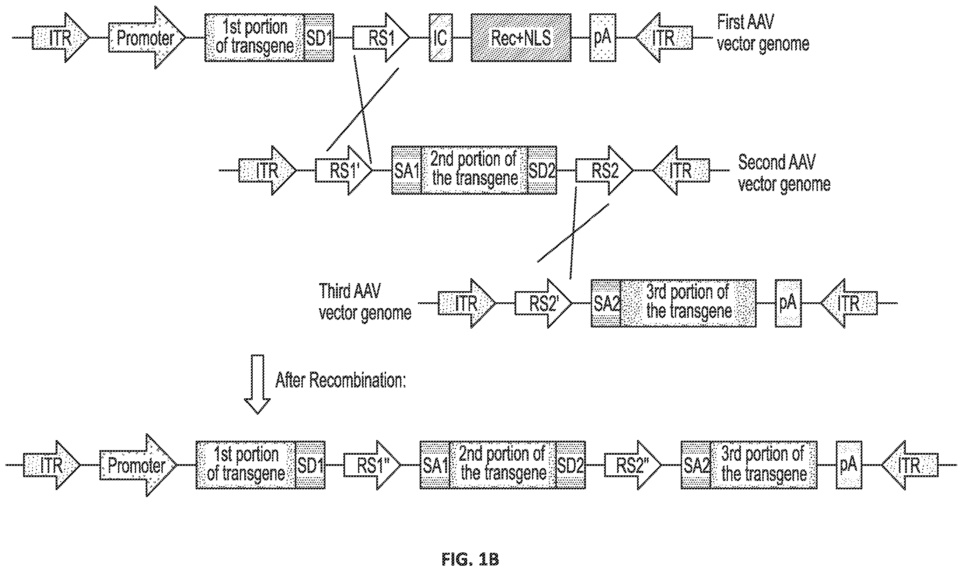

**FIGS. 1A-1B** depict illustrative AAV vector genomes for multipartite gene delivery. FIG. 1A depicts illustrative AAV vector genomes for bipartite gene delivery. "ITR" stands for "inverted terminal repeat"; "SD" stands for "splice donor"; "RS" stands for "recombination site"; "IC" stands for "internal cleavage polypeptide"; "Rec" stands for "recombinase"; "NLS" stands for "nuclear localization sequence"; "pA" stands for "poly-A site"; and "SA" stands for "splice acceptor". FIG. 1B depicts illustrative AAV vector genomes for multipartite gene delivery of a transgene in three portions. "ITR" stands for "inverted terminal repeat"; "SD" stands for "splice donor"; "RS" stands for "recombination site"; "IC" stands for "internal cleavage polypeptide"; "Rec" stands for "recombinase"; "NLS" stands for "nuclear localization sequence"; "pA" stands for "poly-A site"; and "SA" stands for "splice acceptor".
**FIG.** 2 depicts ABCA4 constructs used in tissue culture studies.
**FIG. 3A** shows the necessity of Cre recombinase for expression of full-length ABCA4 protein mediated by recombination of partial ABCA4 constructs transfected into Lec2 cells. FIG. 3B shows the expression level of recombined full-length ABCA4 protein in Lec2 cells transfected with various ABCA4 constructs.
**FIG.** 4 shows the lack of stable expression of auto-cleaved Cre recombinase in Lec2 cells after recombination of partial ABCA4 constructs.
**FIG.** 5 shows the expression level of full-length ABCA4 mRNA in Lec2 cells transfected with various ABCA4 constructs.
**FIG.** 6 shows the expression level of full-length ABCA4 mRNA over time in cells transduced with indicated AAV9 viral particles.

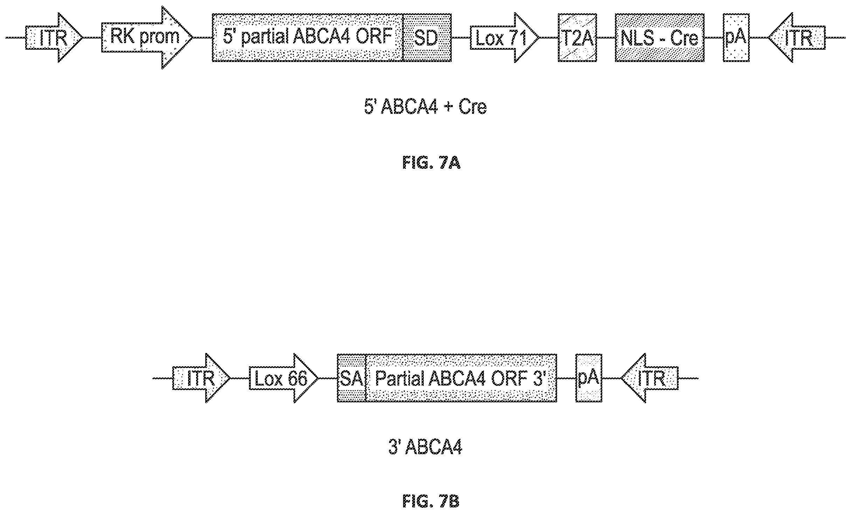

FIGS. 7A-7B depict representative diagrams of AAV vector genomes bipartite delivery of ABCA4 gene. FIG. 7A depicts the construction of a first AAV vector genome (5' ABCA4 + Cre) comprising a human RK promoter, a 5' portion of ABCA4 gene, a Cre recombinase gene and other genomic elements. FIG. 7B depicts the construction of a second AAV vector genome (3' ABCA4) comprising a 3' portion of ABCA4 gene and other genomic elements.

### DETAILED DESCRIPTION

The invention is defined by the claims. Embodiments according to the present disclosure will be described more fully hereinafter.

### Definitions

The practice of the present technology will employ, unless otherwise indicated, conventional techniques of organic chemistry, pharmacology, immunology, molecular biology, microbiology, cell biology and recombinant DNA, which are within the skill of the art. See, e.g., Sambrook, Fritsch and Maniatis, Molecular Cloning: A Laboratory Manual, 2nd edition (1989); Current Protocols In Molecular Biology (F. M. Ausubel, et al. eds., (1987)); the series Methods in Enzymology (Academic Press, Inc.): PCR 2: A Practical Approach (M.J. MacPherson, B.D. Hames and G.R. Taylor eds. (1995)), Harlow and Lane, eds. (1988) Antibodies, a Laboratory Manual, and Animal Cell Culture (RI. Freshney, ed. (1987)).

As used in the description of the invention and the appended claims, the singular forms "a," "an" and "the" are intended to include the plural forms as well, unless the context clearly indicates otherwise.

As used herein, the term "comprising" is intended to mean that the compositions and methods include the recited elements, but do not exclude others. As used herein, the transitional phrase "consisting essentially of" (and grammatical variants) is to be interpreted as encompassing the recited materials or steps and those that do not materially affect the basic and novel characteristic(s) of the recited embodiment. Thus, the term "consisting essentially of" as used herein should not be interpreted as equivalent to "comprising." "Consisting of" shall mean excluding more than trace elements of other ingredients and substantial method steps for administering the compositions disclosed herein. Aspects defined by each of these transition terms are within the scope of the present disclosure.

All numerical designations, e.g., pH, temperature, time, concentration, and molecular weight, including ranges, are approximations which may be varied (+) or (-) by increments of 1.0 or 0.1, as appropriate, or, alternatively, by a variation of +/- 15%, 10%, 5%, 2%. It is to be understood, although not always explicitly stated, that all numerical designations are preceded by the term "about". It also is to be understood, although not always explicitly stated, that the reagents described herein are merely exemplary and that equivalents of such are known in the art. The term "about," as used herein when referring to a measurable value such as an amount or concentration and the like, is meant to encompass variations of 10% of the specified amount.

The terms "acceptable," "effective," or "sufficient" when used to describe the selection of any components, ranges, dose forms, etc. disclosed herein intend that said component, range, dose form, etc. is suitable for the disclosed purpose.

Also, as used herein, "and/or" refers to and encompasses any and all possible combinations of one or more of the associated listed items, as well as the lack of combinations when interpreted in the alternative ("or").

Unless specifically recited, the term "host cell" includes a eukaryotic host cell, including, for example, fungal cells, yeast cells, higher plant cells, insect cells and mammalian cells. Non-limiting examples of eukaryotic host cells include simian, bovine, porcine, murine, rat, avian, reptilian and human, e.g., HEK293 cells and 293T cells.

The term "isolated" as used herein refers to molecules or biologicals or cellular materials being substantially free from other materials.

As used herein, the terms "nucleic acid sequence" and "polynucleotide" are used interchangeably to refer to a polymeric form of nucleotides of any length, either ribonucleotides or deoxyribonucleotides. Thus, this term includes, but is not limited to, single-, double-, or multi- stranded DNA or RNA, genomic DNA, cDNA, DNA-RNA hybrids, or a polymer comprising, consisting essentially of, or consisting of purine and pyrimidine bases or other natural, chemically or biochemically modified, non-natural, or derivatized nucleotide bases.

A "gene" refers to a polynucleotide containing at least one open reading frame (ORF) that is capable of encoding a particular polypeptide or protein. A "gene product" or, alternatively, a "gene expression product" refers to the amino acid sequence (e.g., peptide or polypeptide) generated when a gene is transcribed and translated.

As used herein, "expression" refers to the two-step process by which polynucleotides are transcribed into mRNA and/or the process by which the transcribed mRNA is subsequently translated into peptides, polypeptides, or proteins. If the polynucleotide is derived from genomic DNA, expression may include splicing of the mRNA in a eukaryotic cell.

"Under transcriptional control" is a term well understood in the art and indicates that transcription of a polynucleotide sequence, usually a DNA sequence, depends on its being operatively linked to an element that contributes to the initiation of, or promotes, transcription. "Operatively linked" intends that the polynucleotides are arranged in a manner that allows them to function in a cell. For example, promoters may be operatively linked to the downstream sequences.

The term "encode" as it is applied to polynucleotides refers to a polynucleotide which is said to "encode" a polypeptide if, in its native state or when manipulated by methods well known to those skilled in the art, it can be transcribed to produce the mRNA for the polypeptide and/or a fragment thereof. The antisense strand is the complement of such a nucleic acid, and the encoding sequence can be deduced therefrom.

The term "promoter" as used herein means a control sequence that is a region of a polynucleotide sequence at which the initiation and rate of transcription of a coding sequence, such as a gene or a transgene, are controlled. Promoters may be constitutive, inducible, repressible, or tissue-specific, for example. Promoters may contain genetic elements at which regulatory proteins and molecules such as RNA polymerase and transcription factors may bind. Non-limiting exemplary promoters include a human rhodopsin kinase (RK) promoter, a human interphotoreceptor binding protein promoter (IRBP), a human red/green opsin promoter (pR2.1), a human blue opsin promoter (HB), a mouse opsin promoter (mOP), a mouse short wave opsin promoter (mBP) (short wave opsin promoters may also be referred to as S-opsin or blue opsin promoters), a human rod cGMP phosphodiesterase β-subunit promoter (βPDE), a Rous sarcoma virus (RSV) LTR promoter (optionally with the RSV enhancer), a cytomegalovirus (CMV) promoter, an SV40 promoter, a dihydrofolate reductase promoter, a β-actin promoter, a phosphoglycerol kinase (PGK) promoter, a U6 promoter, an H1 promoter, a ubiquitous chicken β-actin hybrid (CBh) promoter, a small nuclear RNA (U1a or U1b) promoter, an MeCP2 promoter, an MeP418 promoter, an MeP426 promoter, a minimal MeCP2 promoter, a VMD2 promoter, an mRho promoter or an EFI promoter.

Additional non-limiting exemplary promoters provided herein include, but are not limited to EFla, Ubc, human β-actin, CAG, TRE, Ac5, polyhedrin, CaMKIIa, Gal1, TEF1, GDS, ADH1, Ubi, and α-1-antitrypsin (hAAT). It is known in the art that the nucleotide sequences of such promoters may be modified in order to increase or decrease the efficiency of mRNA transcription. See, e.g., Gao et al. (2018) Mol. Ther.: Nucleic Acids 12:135-145 (modifying TATA box of 7SK, U6 and H1 promoters to abolish RNA polymerase III transcription and stimulate RNA polymerase II-dependent mRNA transcription). Synthetically-derived promoters may be used for ubiquitous or tissue specific expression. Further, virus-derived promoters, some of which are noted above, may be useful in the methods disclosed herein, e.g., CMV, HIV, adenovirus, and AAV promoters. In embodiments, the promoter is used together with an enhancer to increase the transcription efficiency. Non-limiting examples of enhancers include an interstitial retinoid-binding protein (IRBP) enhancer, an RSV enhancer or a CMV enhancer.

An enhancer is a regulatory element that increases the expression of a target sequence. A "promoter/enhancer" is a polynucleotide that contains sequences capable of providing both promoter and enhancer functions. For example, the long terminal repeats of retroviruses contain both promoter and enhancer functions. The enhancer/promoter may be "endogenous" or "exogenous" or "heterologous." An "endogenous" enhancer/promoter is one which is naturally linked with a given gene in the genome. An "exogenous" or "heterologous" enhancer/promoter is one which is placed in juxtaposition to a gene by means of genetic manipulation (i.e., molecular biological techniques) such that transcription of that gene is directed by the linked enhancer/promoter. Non-limiting examples of linked enhancer/promoter for use in the methods, compositions and constructs provided herein include a PDE promoter plus IRBP enhancer or a CMV enhancer plus U1a promoter. It is understood in the art that enhancers can operate from a distance and irrespective of their orientation relative to the location of an endogenous or heterologous promoter. It is thus further understood that an enhancer operating at a distance from a promoter is thus "operably linked" to that promoter irrespective of its location in the vector or its orientation relative to the location of the promoter.

The term "protein", "peptide" and "polypeptide" are used interchangeably and in their broadest sense to refer to a compound of two or more subunits of amino acids, amino acid analogs or peptidomimetics. The subunits may be linked by peptide bonds. In another aspect, the subunit may be linked by other bonds, e.g., ester, ether, etc. A protein or peptide must contain at least two amino acids and no limitation is placed on the maximum number of amino acids which may comprise, consist essentially of, or consist of a protein's or peptide's sequence. As used herein the term "amino acid" refers to either natural and/or unnatural or synthetic amino acids, including glycine and both the D and L optical isomers, amino acid analogs and peptidomimetics.

As used herein, the term "signal peptide" or "signal polypeptide" intends an amino acid sequence usually present at the N-terminal end of newly synthesized secretory or membrane polypeptides or proteins. It acts to direct the polypeptide to a specific cellular location, e.g. across a cell membrane, into a cell membrane, or into the nucleus. In embodiments, the signal peptide is removed following localization. Examples of signal peptides are well known in the art. Non-limiting examples are those described in U.S. Patent Nos. 8,853,381, 5,958,736, and 8,795,965. As a further non-limiting example, the signal peptide can be an IDUA signal peptide.

The terms "equivalent" or "biological equivalent" are used interchangeably when referring to a particular molecule, biological material, or cellular material and intend those having minimal homology while still maintaining desired structure or functionality. Non-limiting examples of equivalent polypeptides include a polypeptide having at least about 60%, at least about 65%, at least about 70%, at least about 75%, at least about 80%, at least about 85%, at least about 90%, at least about 95% identity or at least about 99% identity to a reference polypeptide (for instance, a wild-type polypeptide); or a polypeptide which is encoded by a polynucleotide having at least about 70%, at least about 75%, at least about 80%, at least about 85%, at least about 90%, at least about 95% identity, at least about 97% sequence identity or at least about 99% sequence identity to the reference polynucleotide (for instance, a wild-type polynucleotide).

"Homology" or "identity" or "similarity" refers to sequence similarity between two peptides or between two nucleic acid molecules. Percent identity can be determined by comparing a position in each sequence that may be aligned for purposes of comparison. When a position in the compared sequence is occupied by the same base or amino acid, then the molecules are identical at that position. A degree of identity between sequences is a function of the number of matching positions shared by the sequences. "Unrelated" or "non- homologous" sequences share less than 40% identity, less than 25% identity, with one of the sequences of the present disclosure. Alignment and percent sequence identity may be determined for the nucleic acid or amino acid sequences provided herein by importing said nucleic acid or amino acid sequences into and using ClustalW (available at https://genome.jp/tools-bin/clustalw/). For example, the ClustalW parameters can be generated using the Gonnet (for protein) weight matrix. The ClustalW parameters used for performing nucleic acid sequence alignments using the nucleic acid sequences found herein are generated using the ClustalW (for DNA) weight matrix.

As used herein, amino acid modifications may be amino acid substitutions, amino acid deletions or amino acid insertions. Amino acid substitutions may be conservative amino acid substitutions or non-conservative amino acid substitutions. A conservative replacement (also called a conservative mutation, a conservative substitution or a conservative variation) is an amino acid replacement in a protein that changes a given amino acid to a different amino acid with similar biochemical properties (e.g., charge, hydrophobicity or size). As used herein, "conservative variations" refer to the replacement of an amino acid residue by another, structurally or functionally similar residue. Examples of conservative variations include the substitution of one hydrophobic residue such as isoleucine, valine, leucine or methionine for another; or the substitution of one charged or polar residue for another, such as the substitution of arginine for lysine, glutamic acid for aspartic acid, glutamine for asparagine, and the like. Other illustrative examples of conservative substitutions include the changes of: alanine to serine; asparagine to glutamine or histidine; aspartate to glutamate; cysteine to serine; glycine to proline; histidine to asparagine or glutamine; lysine to arginine, glutamine, or glutamate; phenylalanine to tyrosine, serine to threonine; threonine to serine; tryptophan to tyrosine; tyrosine to tryptophan or phenylalanine; and the like.

As used herein, the term "vector" refers to a nucleic acid comprising, consisting essentially of, or consisting of an intact replicon such that the vector may be replicated when placed within a cell, for example by a process of transfection, infection, or transformation. It is understood in the art that once inside a cell, a vector may replicate as an extrachromosomal (episomal) element or may be integrated into a host cell chromosome. Vectors may include nucleic acids derived from retroviruses, adenoviruses, herpesvirus, baculoviruses, modified baculoviruses, papovaviruses, AAV viral vectors, lentiviral vectors, adenovirus vectors, alphavirus vectors and the like. Alphavirus vectors, such as Semliki Forest virus-based vectors and Sindbis virus-based vectors, have also been developed for use in gene therapy and immunotherapy. See, e.g., Schlesinger and Dubensky (1999) Curr. Opin. Biotechnol. 5:434-439 and Ying, et al. (1999) Nat. Med. 5(7):823-827. Exemplary non-viral vectors for delivering nucleic acid include naked DNA; DNA complexed with cationic lipids, alone or in combination with cationic polymers; anionic and cationic liposomes; DNA-protein complexes and particles comprising, consisting essentially of, or consisting of DNA condensed with cationic polymers such as heterogeneous polylysine, defined-length oligopeptides, and polyethyleneimine, in some cases contained in liposomes; and the use of ternary complexes comprising, consisting essentially of, or consisting of a virus and polylysine-DNA.

With respect to general recombinant techniques, vectors that contain both a promoter and a cloning site into which a polynucleotide can be operatively linked are well known in the art. Such vectors are capable of transcribing RNA in vitro or in vivo, and are commercially available from sources such as Agilent Technologies (Santa Clara, Calif) and Promega Biotech (Madison, Wis.). In order to optimize expression and/or in vitro transcription, it may be necessary to remove, add or alter 5' and/or 3' untranslated portions of cloned transgenes to eliminate extra, potential inappropriate alternative translation initiation codons or other sequences that may interfere with or reduce expression, either at the level of transcription or translation. Alternatively, consensus ribosome binding sites can be inserted immediately 5' of the start codon to enhance expression.

As used herein, the term "recombinant expression system" or "recombinant vector" refers to a genetic construct or constructs for the expression of certain genetic material formed by recombination.

A "gene delivery vehicle" is defined as any molecule that can carry inserted polynucleotides into a host cell. Examples of gene delivery vehicles are liposomes, micelles biocompatible polymers, including natural polymers and synthetic polymers; lipoproteins; polypeptides; polysaccharides; lipopolysaccharides; artificial viral envelopes; metal particles; bacteria; viruses, such as baculoviruses, adeno-associated viruses, adenoviruses and retroviruses; bacteriophage, cosmid, plasmid, and fungal vectors; and other recombination vehicles typically used in the art which have been described for expression in a variety of eukaryotic and prokaryotic hosts, and may be used for gene therapy as well as for simple protein expression. Liposomes that also comprise, consist essentially of, or consist of a targeting antibody or fragment thereof can be used in the methods disclosed herein. In addition to the delivery of polynucleotides to a cell or cell population, direct introduction of the proteins described herein to the cell or cell population can be done by the non-limiting technique of protein transfection, alternatively culturing conditions that can enhance the expression and/or promote the activity of the proteins disclosed herein are other non-limiting techniques.

A polynucleotide disclosed herein can be delivered to a cell or tissue using a gene delivery vehicle. "Gene delivery," "gene transfer," "transducing," and the like as used herein, are terms referring to the introduction of an exogenous polynucleotide (sometimes referred to as a "transgene") into a host cell, irrespective of the method used for the introduction. Such methods include a variety of well-known techniques such as vector-mediated gene transfer (by, e.g., viral infection/transfection, or various other protein-based or lipid-based gene delivery complexes) as well as techniques facilitating the delivery of "naked" polynucleotides (such as electroporation, "gene gun" delivery and various other techniques used for the introduction of polynucleotides). The introduced polynucleotide may be stably or transiently maintained in the host cell. Stable maintenance typically requires that the introduced polynucleotide either contains an origin of replication compatible with the host cell or integrates into a replicon of the host cell such as an extrachromosomal replicon (e.g., a plasmid) or a nuclear or mitochondrial chromosome. A number of vectors are known to be capable of mediating transfer of genes to mammalian cells, as is known in the art and described herein.

A "plasmid" is a DNA molecule that is typically separate from and capable of replicating independently of the chromosomal DNA. In many cases, it is circular and doublestranded. Plasmids provide a mechanism for horizontal gene transfer within a population of microbes and typically provide a selective advantage under a given environmental state. Plasmids may carry genes that provide resistance to naturally occurring antibiotics in a competitive environmental niche, or, alternatively, the proteins produced may act as toxins under similar circumstances. It is known in the art that while plasmid vectors often exist as extrachromosomal circular DNA molecules, plasmid vectors may also be designed to be stably integrated into a host chromosome either randomly or in a targeted manner, and such integration may be accomplished using either a circular plasmid or a plasmid that has been linearized prior to introduction into the host cell.

"Plasmids" used in genetic engineering are called "plasmid vectors". Many plasmids are commercially available for such uses. The gene to be replicated is inserted into copies of a plasmid containing genes that make cells resistant to particular antibiotics, and a multiple cloning site (MCS, or polylinker), which is a short region containing several commonly used restriction sites allowing the easy insertion of DNA fragments at this location. Another major use of plasmids is to make large amounts of proteins. In this case, researchers grow bacteria or eukaryotic cells containing a plasmid harboring the gene of interest, which can be induced to produce large amounts of proteins from the inserted gene.

The term "adeno-associated virus" or "AAV" as used herein refers to a member of the class of viruses associated with this name and belonging to the genus Dependoparvovirus, family Parvoviridae. Adeno-associated virus is a single-stranded DNA virus that grows only in cells in which certain functions are provided by a co-infecting helper virus. General information and reviews of AAV can be found in, for example, Carter, 1989, Handbook of Parvoviruses, Vol. 1, pp. 169- 228, and Berns, 1990, Virology, pp. 1743-1764, Raven Press, (New York). It is fully expected that the same principles described in these reviews will be applicable to additional AAV serotypes characterized after the publication dates of the reviews because it is well known that the various serotypes are quite closely related, both structurally and functionally, even at the genetic level. (See, for example, Blacklowe, 1988, pp. 165-174 of Parvoviruses and Human Disease, J. R. Pattison, ed.; and Rose, Comprehensive Virology 3: 1-61 (1974)). For example, all AAV serotypes apparently exhibit very similar replication properties mediated by homologous rep genes; and all bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to "inverted terminal repeat sequences" (ITRs). The similar infectivity patterns also suggest that the replication functions in each serotype are under similar regulatory control. Multiple serotypes of this virus are known to be suitable for gene delivery; all known serotypes can infect cells from various tissue types. At least 11 sequentially numbered naturally-occurring AAV serotypes are known in the art. Non-limiting exemplary serotypes useful in the methods disclosed herein include any of those 11 serotypes, e.g., AAV2, AAV8, AAV9, or variant serotypes, e.g., AAV-DJ and AAV PHP.B. The AAV particle comprises, consists essentially of, or consists of three major viral proteins: VPl, VP2 and VP3. In embodiments, the AAV refers to the serotype AAV1, AAV2, AAV4, AAV5, AAV6, AAV7, AAV8, AAV9, AAV10, AAV11, AAV12, or AAV13. In embodiments, the AAV particle comprises an AAV capsid protein selected from the group consisting of AAVPHP.B, AAVrh74, AAV 110, AAV 204, AAV 214, AAV 214A, AAV 214e, AAV 214e8, AAV 214e9, AAV 214e10, AAV ITB102_45, and AAV 214AB.

An "AAV vector" as used herein refers to a vector comprising one or more heterologous nucleic acid (HNA) sequences and one or more AAV inverted terminal repeat sequences (ITRs). Such AAV vectors can be replicated in a host cell that provides the functionality of rep and cap gene products, and allow the ITRs and the nucleic acid between the ITRs to be packaged into infectious viral particles. In embodiments, AAV vectors comprise a promoter, at least one nucleic acid that may encode at least one protein or RNA, and/or an enhancer and/or a terminator within the flanking ITRs that is packaged into the infectious AAV particle. The ITRs and the nucleic acid between the ITRs may be encapsidated into the AAV caspid, and this encapsidated nucleic acid may be referred to as the "AAV vector genome." AAV vectors may contain elements in addition to the encapsidated portion, for example, antibiotic resistance genes or other elements known in the art included in the plasmid for manufacturing purposes but not packaged into the AAV particle.

As used herein, the term "viral capsid" or "capsid" refers to the proteinaceous shell or coat of a viral particle. Capsids function to encapsidate, protect, transport, and/or release into the host cell a viral genome. Capsids are generally comprised of oligomeric structural subunits of protein ("capsid proteins"). As used herein, the term "encapsidated" means enclosed within a viral capsid. The viral capsid of AAV is composed of a mixture of three viral capsid proteins: VP1, VP2, and VP3. The mixture of VP1, VP2 and VP3 contains 60 monomers that are arranged in a T=1 icosahedral symmetry in a ratio of 1:1:10 (VP1:VP2:VP3) or 1:1:20 (VP1:VP2:VP3) as described in Sonntag F et al., (June 2010). "A viral assembly factor promotes AAV2 capsid formation in the nucleolus". Proceedings of the National Academy of Sciences of the United States of America. 107 (22): 10220-5, and Rabinowitz JE, Samulski RJ (December 2000). "Building a better vector: the manipulation of AAV virions". Virology. 278 (2): 301-8.

An "AAV virion" or "AAV viral particle" or "AAV viral vector" or "AAV vector particle" or "AAV particle" refers to a viral particle composed of at least one AAV capsid protein and an encapsidated polynucleotide from an AAV vector referred to herein as the AAV vector genome.

As used herein, the term "helper" in reference to a virus or plasmid refers to a virus or plasmid used to provide the additional components necessary for replication and packaging of any one of the AAV vector genomes disclosed herein. The components encoded by a helper virus or plasmid may include any genes required for virion assembly, encapsidation, genome replication, and/or packaging. For example, the helper virus or plasmid may encode enzymes or other factors necessary for replication of the viral genome in the host cell. Non-limiting examples of helper viruses and plasmids suitable for use with AAV constructs include pHELP (plasmid), adenovirus (virus), or herpesvirus (virus).

As used herein, a packaging cell (or a helper cell) is a cell used to produce viral vectors. Producing recombinant AAV viral vectors requires AAV Rep and Cap proteins provided in *trans,* as well as adenovirus gene sequences that help AAV replicate. In some aspects, packaging/helper cells contain a plasmid that is stably incorporated into the genome of the cell. In other aspects, the packaging cell may be transiently transfected. Typically, a packaging cell is a eukaryotic cell, such as a mammalian cell or an insect cell.

As used herein, a reporter protein is a detectable protein that is operably linked to a promoter to assay the expression (for example, tissue specificity and/or strength) of the promoter. In aspects, a reporter protein may be operably linked to another polypeptide. In aspects, reporter proteins may be used in monitoring DNA delivery methods, functional identification and characterization of promoter and enhancer elements, translation and transcription regulation, mRNA processing and protein:protein interactions. Non-limiting examples of reporter proteins are β-galactosidase; fluorescent proteins such as green Fluorescent Protein (GFP) or Red Fluorescent Protein (RFP); luciferase; glutathione S-transferase (GST); and maltose binding protein (MBP).

In embodiments, compositions herein may comprise a pharmaceutically acceptable carrier. As used herein, the term "pharmaceutically acceptable carrier" encompasses any of the standard pharmaceutical carriers, such as a phosphate buffered saline solution, water, and emulsions, such as an oil/water or water/oil emulsion, and various types of wetting agents. The compositions also can include stabilizers and preservatives. For examples of carriers, stabilizers and adjuvants, see Martin (1975) Remington's Pharm. Sci., 15th Ed. (Mack Publ. Co., Easton).

A "subject" of diagnosis or treatment is a cell or an animal such as a mammal, or a human. A subject is not limited to a specific species and includes non-human animals subject to diagnosis or treatment and those subject to infections or animal models, including, without limitation, simian, murine, rat, canine, or leporid species, as well as other livestock, sport animals, or pets. In embodiments, the subject is a human.

The term "tissue" is used herein to refer to tissue of a living or deceased organism or any tissue derived from or designed to mimic a living or deceased organism. The tissue may be healthy, diseased, and/or have genetic mutations. The biological tissue may include any single tissue (e.g., a collection of cells that may be interconnected), or a group of tissues making up an organ or part or region of the body of an organism. The tissue may comprise, consist essentially of, or consist of a homogeneous cellular material or it may be a composite structure such as that found in regions of the body including the thorax which for instance can include lung tissue, skeletal tissue, and/or muscle tissue. Exemplary tissues include, but are not limited to those derived from eye, liver, lung, thyroid, skin, pancreas, blood vessels, bladder, kidneys, brain, biliary tree, duodenum, abdominal aorta, iliac vein, heart and intestines, including any combination thereof.

As used herein, "treating" or "treatment" of a disease in a subject refers to (1) preventing the symptoms or disease from occurring in a subject that is predisposed or does not yet display symptoms of the disease; (2) inhibiting the disease or arresting its development; or (3) ameliorating or causing regression of the disease or the symptoms of the disease. As understood in the art, "treatment" is an approach for obtaining beneficial or desired results, including clinical results. For the purposes of the present technology, beneficial or desired results can include one or more, but are not limited to, alleviation or amelioration of one or more symptoms, diminishment of extent of a condition (including a disease), stabilized (i.e., not worsening) state of a condition (including disease), delay or slowing of condition (including disease) progression, amelioration or palliation of the condition (including disease) states and remission (whether partial or total), whether detectable or undetectable.

As used herein the term "effective amount" intends to mean a quantity sufficient to achieve a desired effect. In the context of therapeutic or prophylactic applications, the effective amount may depend on the type and severity of the condition at issue and the characteristics of the individual subject, such as general health, age, sex, body weight, and tolerance to pharmaceutical compositions. In the context of gene therapy, in embodiments an effective amount is an amount sufficient to result in gaining partial or full function of a gene that is deficient in a subject. In other embodiments, the effective amount of an AAV viral particle is the amount sufficient to result in expression of a gene in a subject. The skilled artisan will be able to determine appropriate amounts depending on these and other factors.

In embodiments, the effective amount will depend on the size and nature of the application in question. It will also depend on the nature and sensitivity of the target subject and the methods in use. The skilled artisan will be able to determine the effective amount based on these and other considerations. The effective amount may comprise, consist essentially of, or consist of one or more administrations of a composition depending on the embodiment.

As used herein, the term "administer" or "administration" intends to mean delivery of a substance to a subject such as an animal or human. Administration can be effected in one dose, continuously or intermittently throughout the course of treatment. Methods of determining the most effective means and dosage of administration are known to those of skill in the art and will vary with the composition used for therapy, the purpose of the therapy, as well as the age, health or gender of the subject being treated. Single or multiple administrations can be carried out with the dose level and pattern being selected by the treating physician or in the case of pets and other animals, treating veterinarian.

### AAV Structure and Function

AAV is a replication-deficient parvovirus, the single-stranded DNA genome of which is about 4.7 kb in length, including two 145-nucleotide inverted terminal repeat (ITRs). There are multiple serotypes of AAV. The nucleotide sequences of the genomes of the AAV serotypes are known. For example, the complete genome of AAV-1 is provided in GenBank Accession No. NC_002077; the complete genome of AAV-2 is provided in GenBank Accession No. NC_001401 and Srivastava et al., J. Virol., 45: 555-564 (1983); the complete genome of AAV-3 is provided in GenBank Accession No. NC_001729; the complete genome of AAV-4 is provided in GenBank Accession No. NC_001829; the AAV-5 genome is provided in GenBank Accession No. NC_006152; the complete genome of AAV-6 is provided in GenBank Accession No. NC_001862; the complete genome of AAV-7 is provided in GenBank Accession No. NC_006260; the complete genome of AAV-8 is provided in GenBank Accession No. NC_006261; the AAV-9 genome is provided in Gao et al., J. Virol., 78: 6381-6388 (2004); the AAV-10 genome is provided in Mol. Ther., 13(1): 67-76 (2006); and the AAV-11 genome is provided in Virology, 330(2): 375-383 (2004). The sequence of the AAV rh.74 genome is provided in U.S. Patent 9,434,928. U.S. Patent No. 9,434,928 also provide the sequences of the capsid proteins and a self-complementary genome. In one aspect, the genome is a self-complementary genome. Cis-acting sequences directing viral DNA replication (rep), encapsidation/packaging and host cell chromosome integration are contained within the AAV ITRs. Three AAV promoters (named p5, p19, and p40 for their relative map locations) drive the expression of the two AAV internal open reading frames encoding rep and cap genes. The two rep promoters (p5 and p19), coupled with the differential splicing of the single AAV intron (at nucleotides 2107 and 2227), result in the production of four rep proteins (rep 78, rep 68, rep 52, and rep 40) from the rep gene. Rep proteins possess multiple enzymatic properties that are ultimately responsible for replicating the viral genome.

The cap gene is expressed from the p40 promoter and encodes the three capsid proteins, VPl, VP2, and VP3. Alternative splicing and non-consensus translational start sites are responsible for the production of the three related capsid proteins. More specifically, after the single mRNA from which each of the VP1, VP2 and VP3 proteins are translated is transcribed, it can be spliced in two different manners: either a longer or shorter intron can be excised, resulting in the formation of two pools of mRNAs: a 2.3 kb- and a 2.6 kb-long mRNA pool. The longer intron is often preferred and thus the 2.3-kb-long mRNA can be called the major splice variant. This form lacks the first AUG codon, from which the synthesis of VP1 protein starts, resulting in a reduced overall level of VP1 protein synthesis. The first AUG codon that remains in the major splice variant is the initiation codon for the VP3 protein. However, upstream of that codon in the same open reading frame lies an ACG sequence (encoding threonine) which is surrounded by an optimal Kozak (translation initiation) context. This contributes to a low level of synthesis of the VP2 protein, which is actually the VP3 protein with additional N terminal residues, as is VP1, as described in Becerra SP et al., (December 1985), "Direct mapping of adeno-associated virus capsid proteins B and C: a possible ACG initiation codon", Proceedings of the National Academy of Sciences of the United States of America, 82 (23): 7919-23; Cassinotti P et al., (November 1988), "Organization of the adeno-associated virus (AAV) capsid gene: mapping of a minor spliced mRNA coding for virus capsid protein 1", Virology, 167 (1): 176-84; Muralidhar S et al., (January 1994), "Site-directed mutagenesis of adeno-associated virus type 2 structural protein initiation codons: effects on regulation of synthesis and biological activity", Journal of Virology, 68 (1): 170-6; and Trempe JP, Carter BJ (September 1988), "Alternate mRNA splicing is required for synthesis of adeno-associated virus VP1 capsid protein", Journal of Virology, 62 (9): 3356-63. A single consensus poly-A site is located at map position 95 of the AAV genome. The life cycle and genetics of AAV are reviewed in Muzyczka, Current Topics in Microbiology and Immunology, 158: 97-129 (1992). Non-limiting exemplary capsid proteins include an AAV 110 capsid protein, an AAV 204 capsid protein, an AAV 214 capsid protein, an AAV 214A capsid protein, an AAV 214e capsid protein, an AAV 214e8 capsid protein, an AAV 214e9 capsid protein, an AAV 214e10 capsid protein, an AAV ITB 102 45 capsid protein, an AAV 214AB capsid protein, an AAV6 capsid protein, an AAV8 capsid protein, an AAV9 capsid protein and an AAV2 capsid protein according to Tables 1-3 of the disclosure. Additional non-limiting exemplary capsid proteins include those disclosed in WO2016081811 and, in particular, for AAV capsid protein sequences.

Each VP1 protein contains a VP1 portion, a VP2 portion and a VP3 portion. The VP1 portion is the N-terminal portion of the VP1 protein that is unique to the VP1 protein. The VP2 portion is the amino acid sequence present within the VP1 protein that is also found in the N-terminal portion of the VP2 protein. The VP3 portion and the VP3 protein have the same sequence. The VP3 portion is the C-terminal portion of the VP1 protein that is shared with the VP1 and VP2 proteins.

The VP3 protein can be further divided into discrete variable surface regions I-IX (VR-I-IX). Each of the variable surface regions (VRs) can comprise or contain specific amino acid sequences that either alone or in combination with the specific amino acid sequences of each of the other VRs can confer unique infection phenotypes (e.g., decreased antigenicity, improved transduction and/or tissue-specific tropism relative to other AAV serotypes) to a particular serotype as described in DiMatta et al., "Structural Insight into the Unique Properties of Adeno-Associated Virus Serotype 9" J. Virol., Vol. 86 (12): 6947-6958, June 2012.

AAV has unique features that make it attractive as a vector for delivering foreign DNA to cells, for example, in gene therapy. AAV infection of cells in culture is noncytopathic, and natural infection of humans and other animals is silent and asymptomatic. Moreover, AAV infects many mammalian cell types, allowing the possibility of targeting many different tissues in vivo. Moreover, AAV transduces slowly dividing and non-dividing cells, and can persist essentially for the lifetime of those cells as a transcriptionally active nuclear episome (extrachromosomal element). Furthermore, because the signals directing AAV replication and genome encapsidation are contained within the ITRs of the AAV genome, some or all of the internal approximately 4.3 kb of the genome (encoding replication and structural capsid proteins, rep-cap) may be replaced with foreign DNA to generate AAV vector genomes. The rep and cap proteins may be provided in trans. Another significant feature of AAV is that it is an extremely stable and hearty virus. It easily withstands the conditions used to inactivate adenovirus (56° to 65°C for several hours), making cold preservation of AAV less critical. AAV may even be lyophilized. Finally, AAV-infected cells are not resistant to superinfection.

Multiple studies have demonstrated long-term (> 1.5 years) recombinant AAV-mediated protein expression in muscle. See, Clark et al., Hum Gene Ther, 8: 659-669 (1997); Kessler et al., Proc Nat. Acad Sc. USA, 93: 14082-14087 (1996); and Xiao et al., J Virol, 70: 8098-8108 (1996). See also, Chao et al., Mol Ther, 2:619-623 (2000) and Chao et al., Mol Ther, 4:217-222 (2001). Moreover, because muscle is highly vascularized, recombinant AAV transduction has resulted in the appearance of transgene products in the systemic circulation following intramuscular injection as described in Herzog et al., Proc Natl Acad Sci USA, 94: 5804-5809 (1997) and Murphy et al., Proc Natl Acad Sci USA, 94: 13921- 13926 (1997). Moreover, Lewis et al., J Virol, 76: 8769-8775 (2002) demonstrated that skeletal myofibers possess the necessary cellular factors for correct antibody glycosylation, folding, and secretion, indicating that muscle is capable of stable expression of secreted protein therapeutics. Recombinant AAV (rAAV) genomes of the invention comprise, consist essentially of, or consist of a nucleic acid molecule encoding a therapeutic protein or portion thereof (e.g., the ABCA4) and one or more AAV ITRs flanking the nucleic acid molecule. AAV DNA in the rAAV genomes may be from any AAV serotype for which a recombinant virus can be derived including, but not limited to, AAV serotypes AAV-1, AAV-2, AAV-3, AAV-4, AAV-5, AAV-6, AAV-7, AAV-8, AAV-9, AAV-10, AAV-11, AAV- 12, AAV-13, AAV PHP.B and AAV rh74. Production of pseudotyped rAAV is disclosed in, for example, WO2001083692. Other types of rAAV variants, for example rAAV with capsid mutations, are also contemplated. *See,* e.g., Marsic et al., Molecular Therapy, 22(11): 1900-1909 (2014). The nucleotide sequences of the genomes of various AAV serotypes are known in the art.

### AAV Vector Particles, Capsid Proteins, and AAV Vectors

Provided herein are AAV vector particles, AAV vectors, and capsid proteins that find use in delivering a variety of therapeutic payloads, including nucleic acids and proteins useful in the treatment of disease. The methods and compositions disclosed herein may be used to deliver to a subject transgenes that are larger than the packaging capacity of about 4.7 kb typical of single AAV vector genome. Delivery of the larger transgenes may be achieved by delivering portions of the transgene in two or more AAV vector particles and recombining the portions in a cell to provide a completed transgene (for example, as illustrated in FIG. 1A and FIG. 1B). In a novel preferred approach described herein portions of transgenes are joined by Cre-lox mediated recombination. Advantageously, the design of AAV vector genomes ensures that the 5' portion of the transgene is located upstream of the 3' portion of the transgene after recombination. Recombination sites may also dictate the directionality of the recombination event (*e.g.,* to reduce or eliminate reverse recombination). In a further advantage, the recombination event may also remove the promoter operably linked to the Cre recombinase protein, leaving a promoter-less vector genome comprising the Cre recombinase, thereby preventing further, undesired expression of the Cre recombinase in the host cell.

### AAV Capsid proteins

The disclosure provides AAV particles and AAV vector genomes for multipartite (e.g., bipartite) delivery of large genes. In embodiments, two AAV particles are used for bipartite delivery: a first AAV particle comprises a 5' portion of the gene, and a second AAV particle comprises a 3' portion of the gene. The first and second AAV particles may have the same capsid protein or a different capsid protein.

The capsid protein may comprise, or consist of, any one of the amino acid sequences listed in Table 1, or a sequence having up to 1, 2, 3, 4, 5, 6, 7, 8, 9, or 10 amino acids mutated, deleted or added as compared to, any one of the amino acid sequence listed in Table 1. Optionally, up to 20 amino acids, up to 30 amino acids, or up to 40 amino acids may be mutated, deleted or added compared to these sequences. The capsid protein may be encoded by any one of the nucleic acid sequences listed in Table 1, or a sequence having up to 5, up to 10, up to 30, or up to 60 nucleotide changes to any one of the nucleic acid sequences listed in Table 1.

**Table 1: VP1 Capsid Proteins**

| **Amino Acid SEQ ID NO:** | **Nucleic Acid SEQ ID NO:** | **AAV Capsid Protein Name** |
|---|---|---|
| 1 | 98 | AAV 110 |
| 2 | 15 | AAV 204 |
| 3 | 18 | AAV 214 |
| 30 | 19 | AAV 214A |
| 31 | 20 | AAV 214e |
| 32 | 21 | AAV 214e8 |
| 33 | 22 | AAV 214e9 |
| 34 | 23 | AAV 214e10 |
| 49 | 47 | AAV ITB102_45 |
| 84 | 82 | AAV 214AB |
| 63 | - | AAV6 |
| 67 | 66 | AAV8 |
| 71 | 70 | AAV9 |
| 196 | 197 | AAV2 |
| 205 | - | AAV5 |
| 206 | - | AAV7 |

In embodiments, the AAV capsid protein comprises, consists essentially of, or consists of an amino acid sequence of SEQ ID NOs: 1-3, 30-34, 49, 63, 67, 71, 84, or 196, or a sequence having up to 1, 2, 3, 4, 5, 6, 7, 8, 9, or 10 amino acids different from SEQ ID NOs: 1-3, 30-34, 49, 63, 67, 71, 84, or 196,. Also provided are polynucleotides encoding these VP1 proteins. Polynucleotides encoding the VP1 proteins may comprise, consist essentially of, or consist of the sequence of SEQ ID NOs: 15, 18-23, 47, 66, 70, 82, 98, and 197 or a sequence having up to 5, up to 10, or up to 30 nucleotide changes to SEQ ID NOs: 15, 18-23, 47, 66, 70, 82, 98, and 197.

In embodiments, the AAV capsid protein is an AAV-110 capsid protein (SEQ ID NO: 1), AAV204 capsid protein (SEQ ID NO: 2), AAV214 capsid protein (SEQ ID NO: 3) or AAV ITB 102_45 capsid protein (SEQ ID NO: 49). In embodiments, the AAV capsid protein is a variant of the AAV214 capsid protein. In embodiments, the AAV capsid protein is AAV214A (SEQ ID NO: 30), AAV-214-AB (SEQ ID NO: 84), AAV214e (SEQ ID NO: 31), AAV214e8 (SEQ ID NO: 32), AAV214e9 (SEQ ID NO: 33) or AAV214e10 (SEQ ID NO: 34). In embodiments, the AAV capsid protein is an AAV9 capsid protein (SEQ ID NO: 71.

Sequences for exemplary VP2 and VP3 proteins are provided in Table 2 and Table 3. Given the VP2 and VP3 sequences, the VP1 portions may be determined by alignment with the full, VP1 protein sequence.

**Table 2: VP2 Capsid Proteins**

| **Amino Acid SEQ ID NO:** | **Name** |
|---|---|
| 35 | 214 |
| 36 | 214A |
| 37 | 214e |
| 38 | 214e8 |
| 39 | 214e9 |
| 40 | 214e10 |
| 85 | 214AB |
| 50 | ITB102_45 |

An exemplary nucleic acid for ITB 102_45 is SEQ ID NO: 47. Exemplary nucleic acids for the other capsid VP2 portions may be derived from the corresponding portions of the VP1 capsid protein nucleic acids.

**Table 3: VP3 Capsid proteins**

| **Amino Acid SEQ ID NO:** | **NA SEQ ID NO:** | **AAV Capsid Name** |
|---|---|---|
| 17 | 16 | 204 |
| 41 | 24 | 214 |
| 42 | 25 | 214A |
| 43 | 26 | 214e |
| 44 | 27 | 214e8 |
| 45 | 28 | 214e9 |
| 46 | 29 | 214e10 |
| 86 | 83 | 214AB |
| 51 | 48 | ITB102_45 |

The VP3 proteins of AAV214, AAV214e, AAV214e8, AAV214e9, AAV214e10 have the same amino acid (SEQ ID NO:41) and nucleic acid (SEQ ID NO: 24) sequences.

In embodiments, the AAV VP2 proteins comprise, consist essentially of, or consist of an amino acid sequence of any one of SEQ ID NOs: 35-40, 50 or 85, or a sequence having up to 1, 2, 3, 4, 5, 6, 7, 8, 9, or 10 amino acids different from SEQ ID NOs: 35-40, 50 or 85. Also provided are polynucleotides encoding these VP2 proteins. In embodiments, the polynucleotide encoding the VP2 protein comprises, consists essentially of, or consists of the sequence of SEQ ID NO: 47, or a sequence having up to 5, up to 10, or up to 30 nucleotide changes to SEQ ID NO: 47.

In embodiments, the AAV VP3 proteins comprise, consist essentially of, or consist of an amino acid sequence of SEQ ID NOs: 17, 41-46, 51, or 86, or a sequence having up to 1, 2, 3, 4, 5, 6, 7, 8, 9, or 10 amino acids different from SEQ ID NOs: 17, 41-46, 51, or 86. Also provided are polynucleotides encoding these VP3 proteins. In embodiments, the polynucleotides encoding the proteins that comprise, consist essentially of, or consist of the sequence of SEQ ID NOs: 16, 24-29, 48 and 83, or a sequence having up to 5, up to 10, or up to 30 nucleotide changes to SEQ ID NOs: 16, 24-29, 48 and 83.

In embodiments, the AAV capsid protein is a chimeric protein. In embodiments, a VP1, VP2, or VP3 portion of the AAV capsid protein disclosed herein may be replaced with a VP1, VP2, or VP3 portion from a different AAV capsid protein disclosed herein.

In embodiments, provided herein is an AAV capsid protein comprising a leucine residue at amino acid position 129, an asparagine residue at amino acid position 586 and a glutamic acid residue at amino acid position 723, wherein the amino acid positions in the AAV capsid protein are numbered with respect to amino acid positions in the amino acid sequence of SEQ ID NO: 2. In some cases, the protein comprises the amino acid sequence of SEQ ID NO: 2. In other cases, these amino acids may be introduced into other capsid proteins.

In embodiments, provided herein is an AAV VP1 capsid protein comprising a VP1 portion, a VP2 portion and a VP3 portion, wherein the VP1 portion comprises a leucine (L) residue at amino acid 129, wherein the VP2 portion comprises a threonine (T) or asparagine (N) residue at amino acid 157 and a lysine (K) or serine (S) residue at amino acid 162, and wherein the VP3 portion comprises asparagine (N) residue at amino acid 223, an alanine (A) residue at amino acid 224, a histidine (H) residue at amino acid 272, a threonine (T) residue at amino acid 410, a histidine (H) residue at amino acid 724 and a proline (P) residue at amino acid 734, wherein amino acid positions in the AAV capsid protein are numbered with respect to amino acid positions in the amino acid sequence of SEQ ID NO: 3 (i.e., VP1 capsid subunit numbering).

In embodiments, the VP1 portion further comprises an aspartic acid (D) or alanine (A) residue at amino acid position 24, wherein amino acid positions in the AAV capsid protein are numbered with respect to amino acid positions in the amino acid sequence of SEQ ID NO: 3. In embodiments, the VP2 portion further comprises one or more of (i) a proline (P) residue at amino acid 148; (ii) an arginine (R) residue inserted at amino acid 152; (iii) an arginine (R) residue at amino acid 168; (iv) an isoleucine (I) residue at amino acid 189; and (v) a serine (S) residue at amino acid 200, wherein amino acid positions in the AAV capsid protein are numbered with respect to amino acid positions in the amino acid sequence of SEQ ID NO: 3.

In embodiments, one or more variable regions I through IX in the disclosed VP3 portion capsid proteins may be removed and replaced with alternative regions. Suitable alternatives are identified in Table 4 below. The location for these, as well as the identity of additional alternatives may be identified by alignment to SEQ ID NO:41. In embodiments, one or more VRs may have an insertion of 1, 2 or 3 amino acids. In embodiments, one or more VRs may have a deletion of 1, 2 or 3 amino acids.

**Table 4: VR Alternatives**

| VR | Sequence |
|---|---|
| I | SASTGAS (SEQ ID NO. 52); NSTSGGSS (SEQ ID NO. 53); SSTSGGSS (SEQ ID NO: 87) |
| II | DNNGVK (SEQ ID NO. 54) |
| III | NDGS (SEQ ID NO. 55) |
| IV | INGSGQNQQT (SEQ ID NO. 56) |
| VI | RVSTTTGQNNNSNFAWTA (SEQ ID NO. 57) |
| VII | HKEGEDRFFPLSG (SEQ ID NO. 58); |
| VIII | ADNLQQQNTAPQI (SEQ ID NO. 60); |
| IX | NYYKSTSVDF (SEQ ID NO. 61). |

The disclosure provides nucleic acids encoding any one of the AAV capsid proteins disclosed herein. The disclosure also provides vectors comprising any one of the nucleic acids disclosed herein. The AAV may be an AAV2, an AAV8, or an AAV9 serotype.

### AAV vectors

AAV vectors supply the nucleic acid that becomes encapsidated into the AAV vector particle including elements involved in controlling expression of the nucleic acids in the subject, as well as ITRs to facilitate encapsidation. In embodiments, the AAV vector comprises an AAV vector genome of the disclosure. In embodiments, the AAV vectors disclosed herein comprise at least one heterologous nucleic acid (HNA) sequence or a portion thereof, which, when expressed in a cell of a subject and recombined with the remaining portion of an HNA sequence, encodes a transgene that treats a disease or disorder. Thus, the HNA sequence comprises a transgene, or a portion thereof. In embodiments, the AAV vectors comprise at least one ITR sequence and at least one transgene or portion thereof. In embodiments, the AAV vectors comprise at least one ITR sequence and a portion of one transgene. In embodiments, the transgene encodes a therapeutic protein or a therapeutic RNA.

The present disclosure provides methods of expressing transgenes encoded by genes above a size limit. The size limit is determined by the packaging capacity of the viral particles carrying the heterologous nucleic acid sequence and, for most of AAV viral vectors, the size limit of the encapsidated vector genome is typically about 4.7 kb. The present disclosure provides approaches for delivering to host cells and expressing transgenes that are up to about 8 kb, up to about 7.9 kb, up to about 7.8 kb, up to about 7.7 kb, up to about 7.6 kb, up to about 7.5 kb, or up to about 7.4 kb, using AAV viral particles. The length of the transgene suitable for the bipartite gene delivery approach is between about 4.0 kb to about 8.0 kb; for example between about 4.1 kb to about 7.9 kb, between about 4.2 kb to about 7.8 kb, between about 4.3 kb to about 7.7 kb, between about 4.4 kb to about 7.7 kb, between about 4.5 kb to about 7.7 kb, or between about 4.6 kb to about 7.7 kb. In embodiments, the present disclosure provides multipartite gene delivery approaches for delivering to host cells and expressing transgenes that are up to about 8kb, about 9 kb, about 10 kb, about 11 kb, about 12 kb, about 13 kb, about 14 kb, about 15 kb, about 16 kb, about 17 kb, about 18 kb, about 19 kb, about 20 kb, or any values in between.

Previous approaches for delivering a transgene above the size limit of the AAV system include using homologous recombination mediated by the host cell's own cellular machinery. However, the efficiency of homologous recombination varies widely among different organisms and cell types, and endogenous homologous recombination process is error-prone.

The novel methods described herein employ exogenous recombinases to mediate efficient and precise recombination between the AAV vector genomes encoding different parts of the transgene, which overcomes the problems associated with endogenous homologous recombination. In embodiments, the exogenous recombinase is a Cre recombinase. Cre recombinase belongs to the integrase family of site-specific recombinases. It catalyzes recombination between two recognition sites called Lox sites. A Lox site is typically a 34 bp polynucleotide sequence consisting of a core spacer region of 8 bp and two 13 bp flanking sequences (the recognition regions). The asymmetric core sequence defines an orientation to the Lox site. By using Cre recombination approach methods as disclosed herein, we obtained highly efficient recombination, regardless of the cellular environment and in the absences of additional co-factors. Other exogenous recombinases such as yeast Flippase (Flp) may be used.

The multipartite viral delivery systems of the disclosure use exogenous recombinase (e.g., Cre recombinase) to mediate the recombination and integration of different portions of the transgene of interest via specific recombination sites within the AAV vector genomes that are delivered to the host cell inside the viral vector. In embodiments, three or more portions of a transgene of interest can be delivered using multipartite viral delivery system and form the complete transgene in the host cell using the same strategy.

An illustrative approach for delivering to host cells and expressing a transgene above the size limit of a single AAV vector genome is depicted in FIG. 1A. The transgene is split into two parts: a 5' portion of the transgene, and a 3' portion of the transgene. The 5' portion of the transgene is incorporated into the first AAV vector genome, and the 3' portion of the transgene is incorporated into the second AAV vector genome. The first AAV vector genome also comprises an HNA sequence encoding a recombinase (Rec); for example a Cre recombinase. Once expressed in cells, the recombinase mediates recombination between the first AAV vector genome and the second AAV vector genome through the recombination sites (RS) in the first and second AAV vector genomes, resulting in the formation of two recombined nucleic acids as indicated in **FIG. 1A****.** The first recombined nucleic acid comprises both the 5' and the 3' portions of the transgene which, once transcribed, can be joined together during RNA splicing due to the presence of splice donor (SD) and splice acceptor (SA) sites, resulting in the expression of full-length transgene. The second recombined nucleic acid does not comprise a promoter region, and therefore expression of the recombinase from the second recombined nucleic acid is suppressed.

A first AAV vector genome may comprise, in 5' to 3' orientation:
a. a 5' AAV inverted terminal repeat (ITR);
b. a promoter;
c. the 5' portion of the transgene;
d. a splice donor (SD) site;
e. a recombination site (RS);
f. optionally, a nucleic acid sequence encoding an internal cleavage (IC) polypeptide or an internal ribosome entry site (IRES);
g. a nucleic acid sequence encoding a recombinase, optionally with a nuclear localization sequence (NLS);
h. a poly-A site (pA); and
i. a 3' AAV inverted terminal repeat.

Typically, there is no stop codon between the 5' portion of the transgene and the recombinase nucleic acid, meaning that the HNA region comprising the 5' portion of the transgene and the gene encoding the recombinase (optionally with a nuclear localization sequence) forms a continuous ORF (*i.e.,* are in the same reading frame).

A second AAV vector genome may comprise, in 5' to 3'orientation:
a. a 5' AAV inverted terminal repeat;
b. a recombination site;
c. a splice acceptor (SA) site;
d. the 3' portion of the transgene ORF;
e. a poly-A site;
f. and a 3' AAV inverted terminal repeat.

Elements of the first and second AAV vector genomes facilitate production of the full-length transgene. When both the first AAV vector genome and the second AAV vector genome are present in the same cell, a recombinase is expressed under the control of a promoter in the first AAV vector genome. The recombinase mediates recombination of the first AAV vector genome and the second AAV vector genome via the recombination site in the AAV vector genome and the recombination site in the second AAV vector genome, resulting in the formation of two recombined nucleic acids. The first recombined nucleic acid comprises both the 5' and 3' portions of the transgene, separated by splice donor and acceptor sites. Following transcription and RNA maturation, full-size mRNA is produced from the first recombined nucleic acid. The second recombined nucleic acid comprises the residual nucleic acid portions including the recombinase. Notably the recombinase has little or no expression from the second recombined nucleic acid as it is no longer operably linked to a promoter because the promoter is absent from the nucleic acid. See **FIG. 1A****.**

Three or more portions of the transgene can be delivered to a host cell using a multipartite viral delivery system, and recombined to allow expression of a complete transgene. An illustrative approach for delivering three portions of the transgene is depicted in **FIG. 1B****,** and the same strategy can be used to deliver a transgene using more than three portions. Briefly, in **FIG. 1B****,** a 1st portion of the transgene is incorporated into a first AAV vector genome, a 2nd portion of the transgene is incorporated into a second AAV vector genome, and a 3rd portion of the transgene is incorporated into a third AAV vector genome. The AAV vector genomes are then encapsidated to produce AAV vector particles which are delivered to the host cells and introduce the AAV vector genomes. Similar to the design in **FIG. 1A****,** the first AAV vector genome in **FIG. 1B** comprises a promoter, a first splice donor (SD1) site, a recombination site RS1, optionally a nucleic acid sequence encoding an internal cleavage (IC) polypeptide or an internal ribosome entry site (IRES), a nucleic acid sequence encoding a recombinase (Rec) optionally with a nuclear localization sequence (NLS), and a poly-A site (pA). The second AAV vector genome also comprises a recombination site RS1', a first splice acceptor (SA1) site, a second splice donor (SD2) site, and a recombination site RS2. The third AAV vector genome comprises a recombination site RS2', a second splice acceptor (SA2) site and a poly-A site.

Once the three AAV vector genomes are delivered in the same host cell, the expression of recombinase (e.g., Cre recombinase) mediates the recombination between RS1 and RS1' sites and the recombination between RS2 and RS2' sites. In embodiments, the RS1 and RS1' sites are orthogonal to the RS2 and RS2' sites (i.e., minimal to no recombination between RS1/RS1' and RS2/RS2'). As a non-limiting example, when the recombinase is a Cre recombinase, the RS1 and RS1' can be LoxP sites and the RS2 and RS2' can be LoxN sites. After the recombination, an HNA comprising all three portions of the transgene is formed, and its precursor mRNA transcript can undergo RNA splicing mediated by the SD1/SA1 splicing pair and the SD2/SA2 splicing pair to form a mature mRNA transcript that comprises the integrated transgene without any of the recombination sites. In embodiments, the SD1/SA1 splicing pair and the SD2/SA2 splicing pair are orthogonal to each other (i.e., no splicing occurs between SD1/SA2 pair or SD2/SA1 pair), thereby ensuring that all portions of the transgene are retained in their correct positions within the mature mRNA transcript.

In embodiments, the recombinase is cleaved from the N-terminal portion of the protein via an internal cleavage polypeptide. Expression of the recombinase may be driven by an internal ribosome entry site (IRES).

The first recombined nucleic acid may comprise, in 5' to 3'orientation: a 5' AAV inverted terminal repeat (ITR); a promoter; a 5' portion of the transgene, a splice donor (SD) site; a recombination site (RS) formed by the recombination process; a splice acceptor (SA) site; a 3' portion of the transgene; a poly-A site (pA); and a 3' AAV inverted terminal repeat. The promoter drives efficient transcription of the HNA sequence comprising the transgene, and the 5' portion and 3' portion of the transgene are joined together after splicing of the RNA transcript via the splice donor and acceptor sites, which leads to formation of mRNA transcript of a full-length transgene.

The second recombined nucleic acid may comprise, in 5' to 3'orientation: a 5' AAV inverted terminal repeat (ITR); a recombination site (RS) formed by the recombination process; optionally, an HNA sequence encoding the internal cleavage (IC) polypeptide or an internal ribosome entry site (IRES); a nucleic acid sequence encoding the recombinase, optionally with a nuclear localization sequence (NLS); a poly-A site (pA); and a 3' AAV inverted terminal repeat. In embodiments, the gene encoding the recombinase located in the second recombined nucleic acid cannot be efficiently transcribed due to lack of promoter. As a result, in embodiments, little or no stable expression of the recombinase can be detected in cells transduced with both the first and the second AAV vector genomes.

The recombinase may be a Cre recombinase. The Cre recombinase may comprise, consist essentially of, or consist of, the amino acid sequence of SEQ ID NO: 164 or may have at least about 70%, at least about 75%, at least about 80%, at least about 85%, at least about 90%, at least about 95% identity or at least about 99% identity to SEQ ID NO: 164. Optionally, the Cre recombinase may have 1, 2, 3, 4, 5, 6, 7, 8, 9, or up to 10 amino acids different than SEQ ID NO: 164.

The Cre recombinase may be fused to a nuclear localization sequence (NLS). The location of the NLS may be at the N-terminus, the C-terminus, or in the middle of the Cre recombinase. The NLS may have the amino acid sequence of PKKKRKV (SEQ ID NO: 165), or up to 1, 2, or 3 amino acids variation from SEQ ID NO: 165. The Cre recombinase with NLS may comprise, consist essentially of, or consist of the amino acid sequence of SEQ ID NO: 166 or may have at least about 70%, at least about 75%, at least about 80%, at least about 85%, at least about 90%, at least about 95% identity or at least about 99% identity to SEQ ID NO: 166. The Cre recombinase with NLS maybe encoded by a nucleotide sequence having at least about 70%, at least about 75%, at least about 80%, at least about 85%, at least about 90%, at least about 95% identity, or 100% identity to SEQ ID NO: 167.

The recombination sites recognized by the Cre recombinase may comprise one or more LoxP (locus of X-over P1) sequences. Examples of LoxP sequences are listed in Table 5 below. A representative LoxP sequence is 34 bp in length and comprises an asymmetric 8 bp spacer region in between two sets of 13 bp recognition regions. In embodiments, the LoxP sequence comprises a spacer region having the nucleotide sequence of ATGTATGC. The pairing of recombination sites (one in the first AAV vector genome and the other in the second AAV vector genome) may promote unidirectional recombination and/or prevent backwards recombination.

The recombination site recognized by the Cre recombinase may comprise, consist essentially of, or consist of one or more variant Lox site comprising a spacer region sequence that is different from the canonical sequence (ATGTATGC). In embodiments, the variant Lox site is orthogonal to the LoxP site (i.e., no cross-recombination between the variant Lox site and the LoxP site). In embodiments, the Lox site is a loxN site comprising a spacer region sequence of GTATACCT. In embodiments, the Lox site is a lox2272 site comprising a spacer region sequence of AAGTATCC. In embodiments, the Lox site is a lox511 site comprising a spacer region sequence of ATGTATAC. In embodiments, two or more Lox sites comprising different spacer region sequences are used and these Lox sites are orthogonal to each other. In embodiments, the Lox sites undergo recombination with Lox sites comprising the same spacer region sequence, but are not cross-compatible. More discussions about orthogonal Lox sites can be found in Livet et al. Nature. 2007 Nov 1, 450(7166):56-62 and Missirlis et al. BMC Genomics, 2006 Apr 4, 7:73. Exemplary variant Lox site sequences are provided in Table 5 below.

**Table 5: Exemplary LoxP Sequences and Variant Lox Site Sequences**

| **Name** | **13bp Recognition Region** | **8bp Spacer Region** | **13bp Recognition Region** | **SEQ ID NO** |
|---|---|---|---|---|
| **Wild-Type** | ATAACTTCGTATA | ATGTATGC | TATACGAAGTTAT | SEQ ID NO: 168 |
| **Lox 511** | ATAACTTCGTATA | ATGTATaC | TATACGAAGTTAT | SEQ ID NO: 169 |
| **Lox 5171** | ATAACTTCGTATA | ATGTgTaC | TATACGAAGTTAT | SEQ ID NO: 170 |
| **Lox 2272** | ATAACTTCGTATA | AaGTATcC | TATACGAAGTTAT | SEQ ID NO: 171 |
| **M2** | ATAACTTCGTATA | AgaaAcca | TATACGAAGTTAT | SEQ ID NO: 172 |
| **M3** | ATAACTTCGTATA | taaTACCA | TATACGAAGTTAT | SEQ ID NO: 173 |
| **M7** | ATAACTTCGTATA | AgaTAGAA | TATACGAAGTTAT | SEQ ID NO: 174 |
| **M11** | ATAACTTCGTATA | cgaTAcca | TATACGAAGTTAT | SEQ ID NO: 175 |
| **Lox 71** | TACCGTTCGTATA | NNNTANNN | TATACGAAGTTAT | SEQ ID NO: 176 |
| **Lox 66** | ATAACTTCGTATA | NNNTANNN | TATACGAACGGTA | SEQ ID NO: 177 |
| **Lox 71** | TACCGTTCGTATA | ATGTATGC | TATACGAAGTTAT | SEQ ID NO: 178 |
| **Lox 66** | ATAACTTCGTATA | ATGTATGC | TATACGAACGGTA | SEQ ID NO: 179 |
| **LoxN** | ATAACTTCGTATA | GTATACCT | TATACGAAGTTAT | SEQ ID NO: 207 |

The recombination site in the first AAV vector genome may comprise, consist essentially of, or consist of a Lox 71 sequence; for example, SEQ ID NO: 178, and the recombination site in the second AAV vector genome may comprise, consist essentially of, or consist of a Lox 66 sequence; for example, SEQ ID NO: 179.

The first AAV vector genome may encode an internal cleavage polypeptide to facilitates the cleavage of the recombinase from the rest of the polypeptide. The nucleic acid encoding the internal cleavage polypeptide may be located about 1-1000 bp upstream of the 5' end of the nucleic acid portion encoding the recombinase; for example, about 1-500 bp, about 1-300 bp, about 1-200 bp, about 1-150 bp, about 1-100 bp, about 1-50 bp, about 1-30 bp, about 1-20 bp, or about 1-10 bp upstream of the 5' end of the nucleic acid portion encoding the recombinase.

The internal cleavage polypeptide may be a 2A self-cleaving peptide. Suitable examples of 2A self-cleaving peptide are listed in Table 6 below. Suitable examples include, an internal cleavage peptide having an amino acid sequence of SEQ ID NO: 184, which is encoded by a nucleic acid having the sequence of SEQ ID NO: 185. The C-terminal residues of the internal cleavage peptide may overlap with the N-terminal residues of the Cre recombinase with NLS.

**Table 6: Exemplary 2A Self-cleaving Peptides**

| **Name** | **Sequence** | **SEQ ID NO** |
|---|---|---|
| T2A | EGRGSLLTCGDVEENPGP | SEQ ID NO: 180 |
| P2A | ATNFSLLKQAGDVEENPGP | SEQ ID NO: 181 |
| E2A | QCTNYALLKLAGDVESNPGP | SEQ ID NO: 182 |
| F2A | VKQTLNFDLLKLAGDVESNPGP | SEQ ID NO: 183 |

In embodiments, little or no stable expression of the recombinase can be detected in cells transduced with both the first and the second AAV vector genomes. In embodiments, no expression of the recombinase can be detected by western blot in cells after about 1 hour, about 3 hours, about 6 hours, about 12 hours, about 24 hours, about 48 hours, about 3 days, about 4 days, about 5 days, about 6 days, about 1 week, about 2 weeks, about 3 weeks, or about 1 month, post transduction with both AAV vector genomes. In embodiments, no expression of the recombinase can be detected by western blot in cells after about 48 hours post transduction with both AAV vector genomes. Western blot analysis of the recombinase can be performed by a skilled artisan using commercially available antibodies. For example, the expression level of Cre recombinase can be analyzed by Western blot using rabbit anti-Cre mAb (Cell signaling technology #15036) at 1:10,000 dilution following the protocol described in Example 3.

In embodiments, little or no stable expression of the mRNA encoding the recombinase can be detected in cells transduced with both the first and the second AAV vector genomes. In embodiments, no expression of the mRNA encoding the recombinase can be detected by quantitative PCR (qPCR) in cells after about 1 hour, about 3 hours, about 6 hours, about 12 hours, about 24 hours, about 48 hours, about 3 days, about 4 days, about 5 days, about 6 days, about 1 week, about 2 weeks, about 3 weeks, or about 1 month, post transduction with both AAV vector genomes. In embodiments, no expression of the mRNA encoding the recombinase can be detected by qPCR in cells after about 1 week post transduction with both AAV vector genomes.

The first AAV vector genome may comprise an internal ribosome entry site (IRES) to facilitate expression of a downstream recombinase.

Splice donor (SD) and splice acceptor (SA) sites can be used to remove portions of an mRNA transcript. The splice donor (SD) site may be located downstream of the 5' portion of the transgene in an AAV vector genome. For example, the splice donor (SD) site may be about 1-500 bp, about 1-300 bp, about 1-200 bp, about 1-150 bp, about 1-100 bp, about 1-50 bp, about 1-30 bp, about 1-20 bp, or about 1-10 bp downstream of the 5' portion of the transgene.

The splice acceptor (SA) site may be located upstream of the 3' portion of the transgene in an AAV vector genome. For example, about 1-500 bp, about 1-300 bp, about 1-200 bp, about 1-150 bp, about 1-100 bp, about 1-50 bp, about 1-30 bp, about 1-20 bp, or about 1-10 bp upstream of the 3' portion of the transgene.

The splice donor (SD) site and the splice acceptor (SA) sites may be located within different AAV vector genomes, respectively, such that, after the recombination between the first and the second AAV vector genomes, the mRNA transcript comprising the 5' portion of the transgene and the 3' portion of the transgene can be processed to form a mature mRNA transcript comprising a continuous full-length transgene. See **FIG. 1A****.**

The splice donor (SD) site may comprise, consist essentially of, or consist of the nucleotide sequence of SEQ ID NO: 186, or a sequence having up to 1, up to 3, up to 5, or up to 10 nucleotide changes to SEQ ID NO: 186. The splice acceptor (SA) site may comprise, consist essentially of, or consist of the nucleotide sequence of SEQ ID NO: 187, or a sequence having up to 1, up to 3, up to 5, or up to 10 nucleotide changes to SEQ ID NO: 187.

Control of transgene expression in the host cell may be regulated by regulatory elements contained within the AAV vector genome, including promoter sequences, and poly-A sites. The AAV vector may also encode a signal peptide. In embodiments, the AAV vector genomes have 5' and 3' inverted terminal repeats (ITRs). The 5' ITR is located upstream of a promoter, which in turn is upstream of the transgene. In embodiments, the 5' and 3' ITR have the same sequence. In embodiments, they have a different sequence. In embodiments, an AAV vector genome of the disclosure may comprise, in 5' to 3' orientation, a first (5') ITR, a promoter, a transgene, a poly-A site, and a second (3') ITR.

The HNA (for example, an HNA comprising a transgene or portion thereof) may be operably linked to a constitutive promoter. The constitutive promoter can be any constitutive promoter known in the art and/or provided herein. In embodiments, the constitutive promoter comprises, consists essentially of, or consists of a Rous sarcoma virus (RSV) LTR promoter (optionally with the RSV enhancer), a cytomegalovirus (CMV) promoter, an SV40 promoter, a dihydrofolate reductase promoter, a beta-actin promoter, a phosphoglycerol kinase (PGK) promoter, a U6 promoter, an H1 promoter, a hybrid chicken beta actin promoter, a MeCP2 promotor, an H1 promoter, a U1a promoter, a mMeP418 promoter, a mMeP426 promoter, a minimal MeCP2 promoter, a CAG promoter, or an EF1 promoter. It is known in the art that the nucleotide sequences of such promoters may be modified in order to increase or decrease the efficiency of mRNA transcription. *See, e.g.,* Gao et al. (2018) Mol. Ther.: Nucleic Acids 12:135-145 (modifying TATA box of 7SK, U6 and H1 promoters to abolish RNA polymerase III transcription and stimulate RNA polymerase II-dependent mRNA transcription). In embodiments, the HNA sequence is operably linked to a tissue-specific control promoter, or an inducible promoter. In embodiments, the tissue-specific control promoter is a central nervous system (CNS) cell-specific promoter, a lung-specific promoter, a skin-specific promoter, a muscle-specific promoter, a liver- specific promoter, or an eye-specific promoter. In embodiments, the tissue-specific control promotor comprises, consists essentially of, or consists of an eye-specific promoter. In embodiments, the tissue-specific control promoter comprises, consists essentially of, or consists of a human rhodopsin kinase (RK) promoter, a human interphotoreceptor binding protein promoter (IRBP), a human red/green opsin promoter (pR2.1), a human blue opsin promoter (HB), a mouse opsin promoter (mOP), a mouse short wave opsin promoter (mBP) (short wave opsin promoters may also be referred to as S-opsin or blue opsin promoters), a human rod cGMP phosphodiesterase β-subunit promoter (βPDE), a VMD2 promoter, or an mRho promoter.

A promoter used herein may comprise, consist essentially of or consist of a polynucleotide having the sequence of SEQ ID NO: 96 (mouse U1 promoter) or a SEQ ID NO: 97 (a H1 promoter). The promoter may be a U1a or U1b promoter, EF1 promoter, or CBA (chicken beta-actin). The promoter may be a Human Rhodopsin Kinase (RK) promoter (SEQ ID NO: 188). The promoter may comprise, consist essentially of or consist of any one of the nucleic acid sequences listed in Table 7, or a sequence having up to 5, up to 10, or up to 30 nucleotide changes to any one of the nucleic acid sequences listed in Table 7.

**Table 7:**

| **Promoter name** | **Nucleic acid SEQ ID No.** |
|---|---|
| Mouse U1a promoter | 152 |
| Polymerase III H1 mutant promoter | 153 |
| Chicken β-actin hybrid promoter CBh (CBh promoter consists of CMV enhancer, CBA promoter, first CBA exon and partial intron) | 154 |
| MeCP2 min promoter sequence | 155 |
| MeCP2 promoter sequence | 156 |
| MeCP418 promoter sequence | 157 |
| MeCP426 promoter sequence | 158 |
| VMD2 promoter | 159 |
| PDE6b promoter | 160 |
| mRho promoter | 161 |
| CMV promoter | 162 |
| UbC promoter | 163 |
| Human Rhodopsin Kinase (RK) promoter | 188 |
| Human Interphotoreceptor Binding Protein (IRBP) promoter | 198 |
| Human Red/Green Opsin promoter (pR2.1) | 199 |
| Human Rod cGMP Phosphodiesterase β-subunit promoter (βPDE) | 200 |

In embodiments, the HNA sequence is operably liked to an additional regulatory element. The additional regulatory element can be a woodchuck hepatitis virus post-transcriptional regulatory element ("WPRE"). In embodiments, AAV vectors may comprise regulatory components suitable for growth and culture of the vector in a bacterial host for vector production purposes. For example, the vector may comprise genes for antibiotic resistance, and maintenance of the plasmid in bacteria, as well as associated regulatory elements to control protein expression in bacteria.

In embodiments, the HNA sequence is operably linked to a polyadenylation (poly-A) site. The poly-A site comprises, consists essentially of or consists of an MeCP2 poly-A site, a retinol dehydrogenase 1 (RDH1) poly-A site, a bovine growth hormone (BGH) poly-A site, an SV40 poly-A site, a SPA49 poly-A site (SEQ ID NO: 189), a sNRP-TK65 poly-A site, a sNRP poly-A site, or a TK65 poly-A site. An exemplary SPA49 poly-A sequence is described in Ostedgaard et al., Proc. Nat'l Acad. Sci. USA (Feb. 22, 2005) 102:2952-2957.

### Heterologous nucleic acids (HNA)

The AAV viral vectors disclosed herein infect and deliver one or more heterologous nucleic acids (HNA) to target tissues. The HNA sequences of the first and the second AAV vector genomes are recombined within cells to form new HNA sequences. The HNA sequences are transcribed and, optionally, translated in the cells of the target tissue. In aspects, the new recombinant HNA sequences comprise a transgene encoding a protein.

For transgenes that encode proteins, a transgene encoding the full-length protein is split into two portions, the 5' portion and the 3' portion. The length of each portion is less than the length of the full transgene. The 5' portion of the transgene encodes the N-terminal portion of the protein, and the 3' portion of the transgene encodes the C-terminal portion of the protein. Those skilled in the art will understand that the 5' portion and/or the 3' portion of the transgene does not need to have a nucleic acid length that is the multiple of 3 (*i.e.,* the splitting point of the transgene does not have to be in between two codons), because after the recombination of the first and the second AAV vector genomes, these two portions can be rejoined via mRNA splicing to form the full length transgene. As such, the portion of the transgene and the corresponding amino acid sequence it encodes may be a perfect match or have a small mismatch of 1 or 2 nucleotides at the splitting point. Regarding size limits, the 5' portion of the transgene in a first AAV vector genome may have a size limit of up to about 3.5 kb, about 3.4 kb, about 3.3 kb or about 3.2 kb. The 3' portion of the transgene, in a second AAV vector genome, may have a size limit of up to about 4.6 kb, about 4.5 kb, about 4.4 kb, or about 4.3 kb.

A representative example of a protein encoded by a transgene is ABCA4 (ATP-binding cassette, sub-family A, member 4). Mutations of ABCA4 protein can cause Stargardt disease. In humans, wild-type ABCA4 protein is encoded by the ABCA4 gene and has an amino acid sequence according to Uniprot accession number P78363 (SEQ ID NO: 190). An exemplary nucleic acid sequence encoding the ABCA4 protein is SEQ ID NO: 191.

Suitable ABCA4 proteins may have at least about 70%, at least about 75%, at least about 80%, at least about 85%, at least about 90%, at least about 95% identity, or at least about 99% identity to SEQ ID NO: 190. The ABCA4 protein may be identical to SEQ ID NO: 190, or may have up to 1, 2, 3, 4, 5, 6, 7, 8, 9, or 10 amino acids different from SEQ ID NO: 190.

The nucleic acid encoding the ABCA4 protein is split into a 5' portion and a 3' portions that are put into separate AAV vector genomes. The 5' portion of the ABCA4 transgene may be SEQ ID NO: 192, or a nucleotide sequence having at least about 70%, at least about 75%, at least about 80%, at least about 85%, at least about 90%, at least about 95% identity or at least about 99% identity to SEQ ID NO: 192, and the 3' portion of the ABCA4 transgene may be SEQ ID NO: 193, or a nucleotide sequence having at least about 70%, at least about 75%, at least about 80%, at least about 85%, at least about 90%, at least about 95% identity or at least about 99% identity to SEQ ID NO: 193.

Additional examples of transgenes that are suitable for the bipartite gene delivery method include MYO7A and CEP290.

MYO7A encodes myosin VIIa protein and mutations in MYO7A have been implicated in Usher syndrome. In humans, myosin VIIa protein has an amino acid sequence according to SEQ ID NO: 201. An exemplary nucleic acid sequence of MYO7A gene is SEQ ID NO: 202. Suitable myosin VIIa proteins may have at least about 70%, at least about 75%, at least about 80%, at least about 85%, at least about 90%, at least about 95% identity, or at least about 99% identity to SEQ ID NO: 201. The myosin VIIa protein may be identical to SEQ ID NO: 201, or may have up to 1, 2, 3, 4, 5, 6, 7, 8, 9, or 10 amino acids different from SEQ ID NO: 201.

CEP290 gene encodes a centrosomal protein that is localized to the connecting cilium of the photoreceptors and is involved in both ciliogenesis and ciliary trafficking; mutations in CEP290 have been implicated in Leber congenital amaurosis (LCA). In humans, the CEP290 protein (also known as centrosomal protein of 290 kDa) has an amino acid sequence according to SEQ ID NO: 203. An exemplary nucleic acid sequence of CEP290 gene is SEQ ID NO: 204. Suitable CEP290 proteins may have at least about 70%, at least about 75%, at least about 80%, at least about 85%, at least about 90%, at least about 95% identity, or at least about 99% identity to SEQ ID NO: 203. The CEP290 protein may be identical to SEQ ID NO: 203, or may have up to 1, 2, 3, 4, 5, 6, 7, 8, 9, or 10 amino acids different from SEQ ID NO: 203.

### Methods of Producing AAV Viral Vectors

A variety of approaches may be used to produce AAV viral vectors. In embodiments, packaging is achieved by using a helper virus or helper plasmid and a cell line. The helper virus or helper plasmid contains elements and sequences that facilitate viral vector production. In another aspect, the helper plasmid is stably incorporated into the genome of a packaging cell line, such that the packaging cell line does not require additional transfection with a helper plasmid.

In embodiments, the cell is a packaging or helper cell line. In embodiments In aspects, the helper cell line is eukaryotic cell; for example, an HEK 293 cell or 293T cell. In embodiments, the helper cell is a yeast cell or an insect cell.

A helper plasmid may comprise, for example, at least one viral helper DNA sequence derived from a replication-incompetent viral genome encoding in trans all virion proteins required to package a replication incompetent AAV, and for producing virion proteins capable of packaging the replication-incompetent AAV at high titer, without the production of replication-competent AAV.

Helper plasmids for packaging AAV are known in the art, see, e.g., U.S. Patent Pub. No. 2004/0235174 A1. As stated therein, an AAV helper plasmid may contain as helper virus DNA sequences, by way of non-limiting example, the Ad5 genes E2A, E4 and VA, controlled by their respective original promoters or by heterologous promoters. AAV helper plasmids may additionally contain an expression cassette for the expression of a marker protein such as a fluorescent protein to permit the simple detection of transfection of a desired target cell.

The disclosure provides methods of producing AAV particles comprising transfecting a packaging cell line with any one of the AAV helper plasmids disclosed herein; and any of the AAV vectors disclosed herein. In embodiments, the AAV helper plasmid and AAV vector are co-transfected into the packaging cell line. In embodiments, the cell line is a mammalian cell line, for example, human embryonic kidney (HEK) 293 cell line. The disclosure provides cells comprising any of the AAV vectors, AAV vector genomes, and/or AAV particles disclosed herein.

### Pharmaceutical compositions

The disclosure provides pharmaceutical compositions comprising any of the AAV vectors, AAV vector genomes, AAV capsids and/or AAV particles described herein. Typically, the AAV particles are administered for therapy.

The pharmaceutical composition, as described herein, may be formulated by any methods known or developed in the art of pharmaceutical drug development, which methods include but are not limited to contacting the active ingredients (e.g., viral particles or recombinant vectors) with an excipient or other accessory ingredient, dividing or packaging the product to a dose unit. In embodiments, the pharmaceutical composition is suitable for ocular injection.

In embodiments, the pharmaceutical composition may further comprise saline, lipidoids, liposomes, lipid nanoparticles, polymers, lipoplexes, core-shell nanoparticles, peptides, proteins, cells transduced with viral vectors (e.g., for transplantation into a subject), nanoparticle mimics or combinations thereof. In embodiments, the pharmaceutical composition is formulated as a nanoparticle. In embodiments, the pharmaceutical composition is formulated as sterile and substantially isotonic solution.

A pharmaceutical composition in accordance with the present disclosure may be prepared, packaged, and/or sold in bulk, as a single unit dose, and/or as a plurality of single unit doses. The amount of the active ingredient is generally equal to the dosage of the active ingredient which would be administered to a subject and/or a convenient fraction of such a dosage such as, for example, one -half or one-third of such a dosage. The formulations of the invention can include one or more excipients, each in an amount that together increases the stability of the viral vector, increases cell transfection or transduction by the viral vector, increases the expression of vector genome encoded protein, and/or alters the release profile of vector genome encoded proteins. In embodiments, the pharmaceutical composition comprises an excipient. Non limiting examples of excipients include solvents, dispersion media, diluents, or other liquid vehicles, dispersion or suspension aids, surface active agents, isotonic agents, thickening or emulsifying agents, preservatives, or combination thereof.

In embodiments, the pharmaceutical composition comprises a cryoprotectant. The term "cryoprotectant" refers to an agent capable of reducing or eliminating damage to a substance during freezing. Non-limiting examples of cryoprotectants include sucrose, trehalose, lactose, glycerol, dextrose, raffinose and/or mannitol.

### Therapeutic Methods

This disclosure provides methods of preventing or treating a genetic disorder, comprising, consisting essentially of, or consisting of administering to a subject a therapeutically effective amount of the pharmaceutical compositions disclosed herein.

In embodiments, the genetic disorder is an eye disorder, a CNS disorder, a skin disorder, a lung disorder, a muscle disorder, a liver disorder, a digestive disorder, a blood or lymphatic disorder, an inflammatory disorder, or a cancer. In embodiments, the disorder is Stargardt disease.

In embodiments, the disorder is hypophosphatasia, amyotrophic lateral sclerosis (ALS), spinal muscular atrophy (SMA), recessive dystrophic epidermolysis bullosa (RDEB), lysosomal storage disorder (including Duchenne's Muscular Dystrophy, and Becker muscular dystrophy), juvenile Batten disease, infantile Batten disease, autosomal dominant disorders, muscular dystrophy, Bietti's Crystalline Dystrophy, retinoschisis (e.g., degenerative, hereditary, tractional, exudative), hemophilia A, hemophilia B, multiple sclerosis, diabetes mellitus, Fabry disease, Pompe disease, neuronal ceroid lipofuscinosis 1 (CLN1), CLN3 disease (or juvenile neuronal ceroid lipofuscinosis), Gaucher disease, cancer, arthritis, muscle wasting, heart disease, intimal hyperplasia, Rett syndrome, epilepsy, Huntington's disease, Parkinson's disease, Alzheimer's disease, an autoimmune disease, cystic fibrosis, thalassemia, Hurler's Syndrome (MPS IH), Sly syndrome, Scheie Syndrome, Hurler-Scheie Syndrome, Hunter's Syndrome, Sanfilippo Syndrome A (mucopolysaccharidosis IIIA or MPS IIIA), Sanfilippo Syndrome B (mucopolysaccharidosis IIIB or MPS IIIB), Sanfilippo Syndrome C, Sanfilippo Syndrome D, Morquio Syndrome, Maroteaux-Lamy Syndrome, Krabbe's disease, phenylketonuria, spinal cerebral ataxia, LDL receptor deficiency, hyperammonemia, anemia, arthritis, or adenosine deaminase deficiency.

In addition to specific transgenes disclosed herein, known active enzymes, structural proteins, or RNA sequences may be used as transgenes to deliver functional activity.

The disorder may be an ophthalmic disease. The eye is immune privileged tissue and a low dose of viruses may provide a therapeutic benefit. The ophthalmic disease may affect photoreceptor and/or RPE cells. For example, the ophthalmic disease may be retinitis pigmentosa (e.g., autosomal recessive (SPATA7 gene; LRAT gene; TULP1 gene), autosomal dominant (AIPL1 gene), and X-linked (RPGR gene)), an eye disorder related to mutations in the bestrophin-1 (BEST-1) gene (e.g., vitelliform macular dystrophy, age-related macular degeneration, autosomal dominant vitreoretinochoroidopathy, glaucoma, cataracts), Leber congenital amaurosis (LCA; aryl-hydrocarbon interacting protein-like 1 (AIPL1) gene), cone-rod dystrophy (CRD; ABCA4 gene), Stargardt's (ABCA4 gene), choroideremia (CHM gene), Usher Syndrome (MYO7A gene; CDH23 gene; USH2A gene; CLRN1 gene), retinoschisis (RS1 gene), Bietti's Crystalline Dystrophy (CYP4V2 gene) or Achromatopsia (CNGA3 gene, CNGB3 gene, GNAT2 gene, PDE6C gene, or PDE6H gene).

Suitable disorders to treat using the methods and compositions of the disclosure include Usher syndrome (USH) and Leber congenital amaurosis (LCA). Usher syndrome is an autosomal recessive disorder characterized by hearing impairment associated with retinitis pigmentosa and in some cases vestibular dysfunction. Usher syndrome type I (USH1) is the most serious type, characterized by severe to profound congenital sensorineural hearing loss, balance deficiency and prepubertal onset of retinitis pigmentosa leading to blindness. Six loci for USH1 (USH1B-USH1G) have been mapped. Mutations in MYO7A gene were found to be responsible for USH1B which is the most common subtype of USH1. MYO7A gene encodes myosin VIIA protein. In the human retina, myosin VIIA displays an active function in the migration of RPE melanosomes, phagocytosis of photoreceptor cell outer segment tips and opsin transport through the cilium of these photoreceptors. The coding sequence of MYO7A has a size of ~6.6 kb and the encoded myosin VIIA has an amino acid sequence according to Uniprot accession number Q13402-1 (www.uniprot.org/uniprot/Q13402#Q13402-1). Leber congenital amaurosis (LCA) refers to a group of severe inherited retinal disorders characterized by poor vision within the first year of life, nystagmus, and a non-recordable electroretinogram. Mutations in the CEP290 are one of the most common contributors to LCA. CEP290 gene encodes a centrosomal protein that is localized to the connecting cilium of the photoreceptors and is involved in both ciliogenesis and ciliary trafficking. CEP290 plays a critical role in primary cilium formation and ciliary protein transport. Patients with CEP290-associated LCA retain some cone nuclei in the central cone-rich foveal region. However, these cone photoreceptors have abnormal inner and outer segments resulting in severe vision loss in most patients. The coding sequence of CEP290 gene has a size of ~7.4kb and encodes a protein sequence according to Uniprot accession number 015078-1 (www.uniprot.org/uniprot/O15078#O15078-1).

In embodiments, the cancer is a solid cancer; for example, bladder, breast, cervical, colon, rectal, endometrial, kidney, lip, oral, liver, melanoma, mesothelioma, non-small cell lung, non-melanoma skin, ovarian, pancreatic, prostate, sarcoma, small cell lung tumor, or thyroid.

The subject may be a mammal; for example, a human. The human may be an infant human; for example, under 3 years old, 2 years old, or under 1 year old.

The methods of treatment and prevention disclosed herein may be combined with appropriate diagnostic techniques to identify and select patients for the therapy or prevention. For example, the method of treating or preventing a disorder, for example, Stargardt disease, disclosed herein may further comprise steps of performing a genetic test to identify a gene mutation or deletion related to the disorder in the subject. In embodiments, the method of treating or preventing a disorder, for example, Stargardt disease, comprises administering to a subject who has been previously identified as carrying a mutation related to the disorder, or as being at high risk for developing the disorder (for example, based on hereditary factors).

The disclosure provides methods of increasing the level of a protein in a host cell, comprising contacting the host cell with two AAV viral particles as disclosed herein, wherein the first AAV viral particle comprises a first AAV vector genome, and the second AAV viral particle comprises a second AAV vector genome. Cre-lox mediated recombination of the first AAV vector genome and the second AAV vector genome results in an HNA sequence encoding the protein. In embodiments, the protein is a therapeutic protein. In embodiments, the host cell is *in vitro, in vivo,* or *ex vivo.* In embodiments, the host cell is derived from a subject. In embodiments, the subject suffers from a disorder, which results in a reduced level and/or functionality of the protein, as compared to the level and/or functionality of the protein in a normal subject.

The disclosure provides methods of preventing or treating a disorder of a subject by introducing a transgene into the subject. In embodiments, the method comprises co-administration of effective amounts of two AAV viral particles - the first AAV viral particle comprises a first AAV vector genome comprising a 5' portion of the transgene, and the second AAV viral particle comprises a second AAV vector genome comprising a 3' portion of the transgene. For example, the disclosure provides methods of treating Stargardt disease in a subject. In embodiments, the methods comprise co-administration of two AAV viral particles - the first AAV viral particle comprises the first AAV vector genome comprising a 5' portion of an ABCA4 gene, and the second AAV viral particle comprises the second AAV vector genome comprising a 3' portion of an ABCA4 gene.

### Dosage and Administration

Methods of determining the most effective means and dosage of administration are known to those of skill in the art and will vary with the composition used for therapy, the purpose of the therapy and the subject being treated. Single or multiple administrations can be carried out with the dose level and pattern being selected by the treating physician. It is noted that dosage may be impacted by the route of administration. Suitable dosage formulations and methods of administering the agents are known in the art. Non-limiting examples of such suitable dosages may be as low as 10⁹ vector genomes to as much as 10¹⁷ vector genomes per administration.

In embodiments of the methods described herein, the number of viral particles (e.g., AAV) administered to the subject ranges from about 10⁹ to about 10¹⁷. In particular some embodiments, about 10¹⁰ to about 10¹², about 10¹¹ to about 10¹³, about 10¹¹ to about 10¹², about 10¹¹ to about 10¹⁴, about 5 x 10¹¹ to about 5 x 10¹², or about 10¹² to about 10¹³ viral particles are administered to the subject. For administration to a human eye, a total dose of about 1 x 10¹⁰ vg/eye may be used, and a total dose of 5 x 10⁹ vg/eye may be used for a mouse eye. Non-invasive, in vivo imaging techniques can be used to monitor efficacy/safety in animals, which include but are not limited to scanning laser ophthalmoscopy (SLO), optical coherence tomography (OCT), multi-photon microscopy, fluorescein angiography.

In embodiments, the viral particle is introduced to the subject intravenously, intrathecally, intracerebrally, intraventricularly, intranasally, intratracheally, intra-aurally, intra-ocularly, or peri-ocularly, orally, rectally, transmucosally, inhalationally, transdermally, parenterally, subcutaneously, intradermally, intramuscularly, intrapleurally, topically, intralymphatically, intracisternally; such introduction may also be intra-arterial, intracardiac, subventricular, epidural, intracerebral, intracerebroventricular, sub-retinal, intravitreal, intraarticular, intraperitoneal, intrauterine, or any combination thereof. In embodiments, the viral particles are delivered to a desired target tissue, e.g., to the lung, eye, or CNS, as non-limiting examples. In embodiments, delivery of viral particles is systemic. The intracisternal route of administration involves administration of a drug directly into the cerebrospinal fluid of the brain ventricles. It could be performed by direct injection into the cisterna magna or via a permanently positioned tube.

For treating an ophthalmic disease (an eye disorder) intraocularly, there are multiple modes of administration known to those skilled in the art, including but not limited to: lacrimal gland (LG) administration, topical eye drop, intra-stromal administration to the cornea, intra-cameral administration (anterior chamber), intravitreal administration, sub-retinal administration, systemic administration, or a combination thereof. 80% of genetic eye disorders occur in the photoreceptors. Intravitreal delivery of small volume gene therapies can occur in an out-patient clinic.

Some AAV particles show tropism for brain and cervical spine and may also cross the blood-brain-barrier (BBB). Some AAV particles show high retinal tropism by subretinal and intravitreal injections. AAV particles of may target multiple eye cell types, such as, for example, cones, rods, and retinal pigment epithelium (RPE). Advantageously, AAV particles may escape neutralizing antibodies against natural serotypes, and thus enable potential redosing. In a further aspect, AAV particles and compositions may be administered in combination with other known treatments for the disorder being treated.

The AAV particles may be administered via subretinal injection. Routes of subretinal injection comprises: (1) a transcorneal route through the pupil, passing the lens, vitreous, and retina; (2) a transscleral route entering the pars plana or limbus areas, crossing through the vitreous and the opposite side of retina into the subretinal space; and (3) a transscleral route through the choroid and Bruch's membrane without penetrating the retina. Subretinal injection is described in Peng et al. Ophthalmic Res. 2017;58(4):217-226.

Administration of each of the AAV vectors, AAV viral particles or compositions of this disclosure can be effected in one dose, continuously or intermittently throughout the course of treatment. In embodiments, the AAV vectors, AAV particles or compositions of this disclosure are parenterally administered by injection, infusion or implantation.

An AAV viral particle comprising the first AAV vector genome and an AAV viral particle comprising the second AAV vector genome may be co-administered or administered sequentially. "Co-administration" refers to administration of a first and a second AAV viral particle together *(i.e.* both AAV viral particles may be administered in the same composition). "Sequential administration" means that two AAV viral particles are administered separately but within a time interval such that they can act together on the target cells and achieve a physiological effect (i.e., transduce the same cell in a subject and recombine the transgene portions to produce of full-length transgene). For example, administration of one viral particle can precede administration of the other viral particle by up to about 10 minutes, up to about 20 minutes, up to about 30 minutes, up to about 60 minutes, up to about 2 hours, up to about 3 hours, up to about 6 hours, up to about 12 hours, up to about 24 hours, up to about 2 days, up to about 4 days, up to about 1 week, up to about 2 weeks, or up to about 4 weeks.

The AAV viral particle comprising the first AAV vector genome and the AAV viral particle comprising the second AAV vector genome may be administered at a specific ratio or within a specific range of ratios. In embodiments, the ratio of the AAV viral particle comprising the first AAV vector genome to the AAV viral particle comprising the second AAV vector genome is about 0.1:1, about 0.2:1, about 0.3:1, about 0.4:1, about 0.5:1, about 0.6:1, about 0.7:1, about 0.8:1, about 0.9:1, about 1:1, about 1.1:1, about 1.2:1, about 1.3:1, about 1.5:1, about 1.7:1, about 2:1, about 3:1, about 4:1, about 5:1, about 6:1, about 7:1, about 8:1, about 9:1, or about 10:1. In embodiments, the ratio of the AAV viral particle comprising the first AAV vector genome to the AAV viral particle comprising the second AAV vector genome is within the range of 0.1:1 to 10:1, 0.2:1 to 5:1, 0.3:1 to 3:1, 0.4:1 to 2.5:1, 0.5:1 to 2:1, 0.7:1 to 1.5:1, 0.8:1 to 1.2:1, or 0.9:1 to 1.1:1. In embodiments, the ratio of the AAV viral particle comprising the first AAV vector genome to the AAV viral particle comprising the second AAV vector genome is less than 0.1:1, less than 0.2:1, less than 0.3:1, less than 0.4:1, less than 0.5:1, less than 0.6:1, less than 0.7:1, less than 0.8:1, less than 0.9:1, or less than 1:1. In embodiments, the ratio of the AAV viral particle comprising the first AAV vector genome to the AAV viral particle comprising the second AAV vector genome is about 1:1. In embodiments, the ratio is calculated by comparing the total number of the AAV viral particle comprising the first AAV vector genome to the total number of the AAV viral particle comprising the second AAV vector genome during the course of administration (e.g., co-administration or sequential administration).

### Kits

The agents, vectors, or compositions described herein may, in embodiments, be assembled into pharmaceutical or diagnostic or research kits to facilitate their use in therapeutic, diagnostic or research applications. In embodiments, the kits of the present disclosure include any one of the modified AAV capsid proteins, AAV vectors, AAV vector genomes, AAV particles, host cells, isolated tissues, compositions, or pharmaceutical compositions as described herein.

A kit may further comprise instructions for use. Specifically, such kits may include one or more agents described herein, along with instructions describing the intended application and the proper use of these agents. As an example, In embodiments, the kit may include instructions for mixing one or more components of the kit and/or isolating and mixing a sample and applying to a subject. In embodiments, agents in a kit are in a pharmaceutical formulation and dosage suitable for a particular application and for a method of administration of the agents. Kits for research purposes may contain the components in appropriate concentrations or quantities for running various experiments.

The kit may be designed to facilitate use of the methods described herein and can take many forms. Each of the compositions of the kit, where applicable, may be provided in liquid form (e.g., in solution), or in solid form, (e.g., a dry powder). In certain cases, some of the compositions may be constitutable or otherwise processable (e.g., to an active form), for example, by the addition of a suitable solvent or other species (for example, water or a cell culture medium), which may or may not be provided with the kit.

In embodiments, the kit contains any one or more of the components described herein in one or more containers. Thus, in embodiments, the kit may include a container housing agents described herein. The agents may be in the form of a liquid, gel or solid (powder). The agents may be prepared sterilely, packaged in a syringe and shipped refrigerated. Alternatively, they may be housed in a vial or other container for storage. A second container may have other agents prepared sterilely. Alternatively, the kit may include the active agents premixed and shipped in a syringe, vial, tube, or other container. The kit may have one or more or all of the components required to administer the agents to a subject, such as a syringe, topical application devices, or IV needle tubing and bag.

It is to be understood that while the invention has been described in conjunction with the above embodiments, the foregoing description and examples are intended to illustrate and not limit the scope of the invention. Other aspects, advantages and modifications within the scope of the invention will be apparent to those skilled in the art to which the invention pertains.

### EXAMPLES

### EXAMPLE 1:

### Construction of Plasmids with Bipartite Design

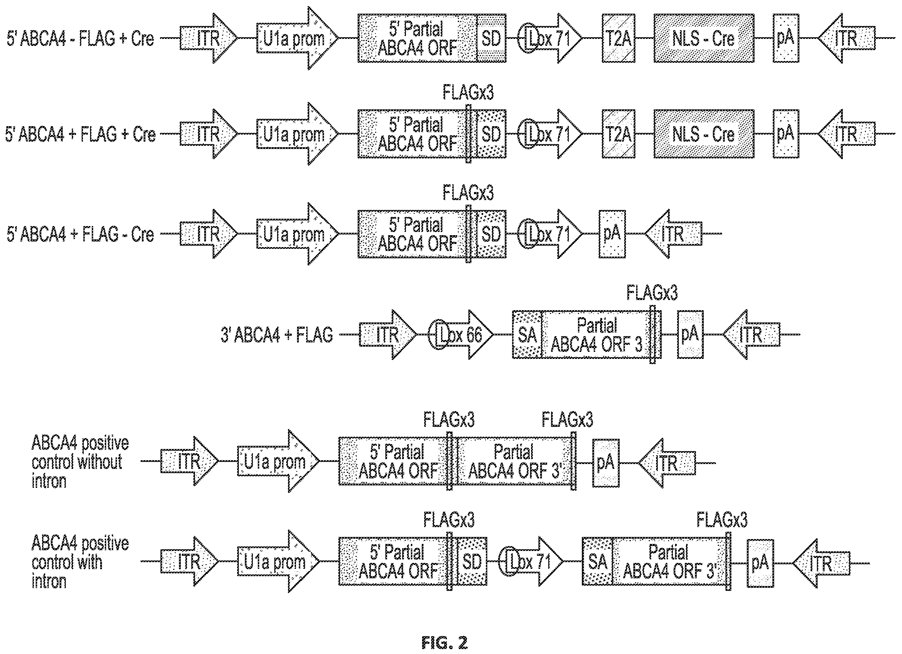

Several plasmids were generated to test the expression of full length ABCA4 gene in cells using the bipartite vector system **(****FIG. 2****).**

A "5' ABCA4 -FLAG +Cre" plasmid was constructed, which comprises, from 5' to 3': 5' AAV inverted terminal repeat, a U1a promoter, a 5' portion of the ABCA4 gene ORF, a splice donor (SD) site, a Lox71 site, the nucleic acid sequence encoding T2A peptide, the nucleic acid sequence encoding Cre recombinase with nuclear localization sequence (NLS), a poly-A site, and 3' AAV inverted terminal repeat.

A "5' ABCA4 +FLAG +Cre" plasmid was generated by incorporating a 3xFLAG tag coding sequence into the 5' portion of the ABCA4 gene in the "5' ABCA4 -FLAG +Cre" plasmid.

A "5' ABCA4 +FLAG -Cre" plasmid was generated by removing the Cre recombinase coding sequence in the "5' ABCA4 +FLAG +Cre" plasmid.

A "3' ABCA4 +FLAG" plasmid was constructed, which comprises, from 5' to 3': 5' AAV inverted terminal repeat, a Lox66 site, a splice acceptor (SA) site, 3' portion of the ABCA4 gene ORF incorporated with a 3xFLAG tag coding sequence, a poly-A site, and a 3' AAV inverted terminal repeat.

Two positive control plasmid constructs comprising the full length ABCA4 gene were also generated **(****FIG. 2****).** One positive control construct has no intron region, whereas the other positive control comprises an intron region which comprises a splice donor (SD) site, a Lox71 site, and a splice acceptor (SA) site, mimicking the construct generated by the recombination between "5' ABCA4 +FLAG +Cre" and "3' ABCA4 +FLAG" plasmid. Both positive control constructs comprise 3xFLAG tag coding sequences in both the 5' portion of the ABCA4 gene and the 3' portion of the ABCA4 gene.

### EXAMPLE 2:

### Expression of Full-Length Protein Using a Bipartite Vector System

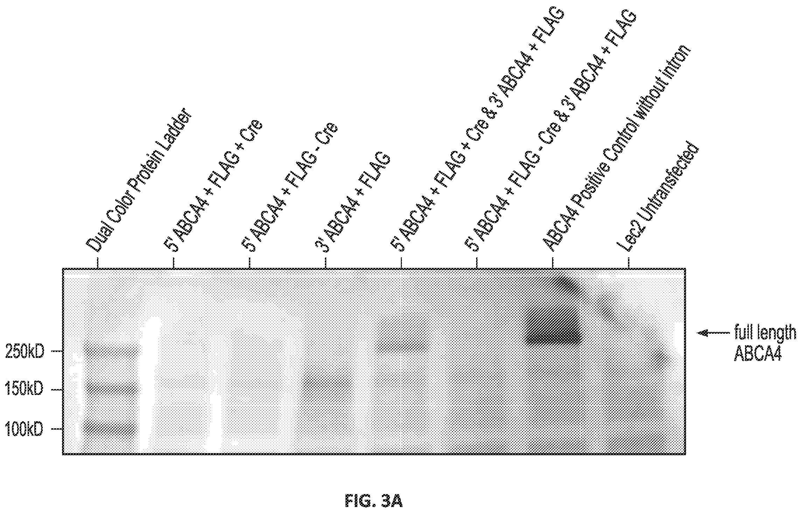

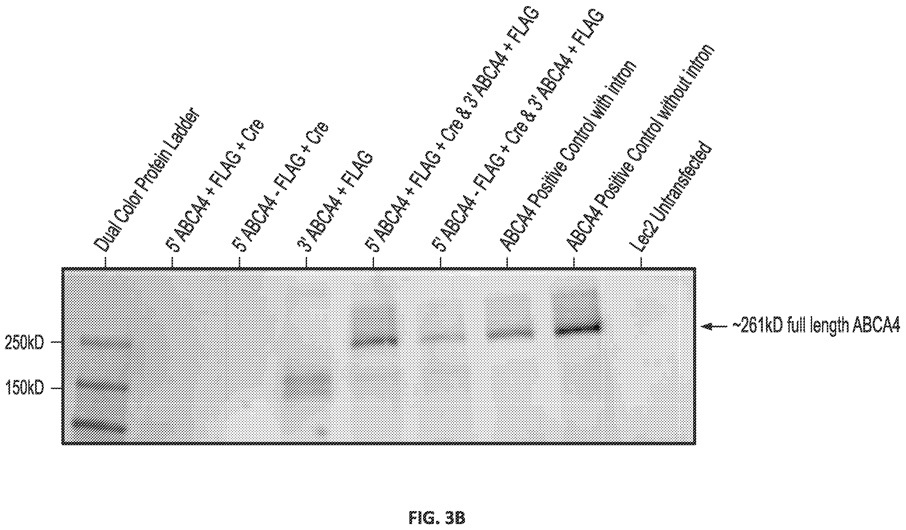

Cell studies were performed using Lec2 cells transfected with the indicated plasmids described in Example 1 and **FIG. 2****,** and the expression of full length ABCA4 protein was examined using Western blotting. Full length ABCA4 protein (with attached FLAG tags) has an estimated molecular weight of ~261 kD. As shown in **FIG. 3A****,** among the cells that were transfected with the plasmids other than the positive control, expression of full length ABCA4 protein was only observed when both "5' ABCA4 +FLAG +Cre" and "3' ABCA4 +FLAG" plasmids were applied to the cells. On the other hand, there was no expression of full length ABCA4 protein in cells transfected with the dual vector system without Cre recombinase (i.e., the "5' ABCA4 +FLAG -Cre" and "3' ABCA4 +FLAG" pair). Similarly, in a repeat experiment **(****FIG. 3B****),** among the cell groups that were transfected with the indicated plasmids other than the positive controls, only two experimental groups showed expression of full length ABCA4. One group was transfected with "5' ABCA4 +FLAG +Cre" and "3' ABCA4 +FLAG" plasmids, and the other group was transfected with "5' ABCA4 -FLAG +Cre" and "3' ABCA4 +FLAG" plasmids. These results indicate that the dual vector system can carry out efficient Cre-lox mediated recombination between the two plasmids which in turn results in expression of the full-length ABCA4 protein.

### EXAMPLE 3:

### No Detectable Expression of Cre Recombinase from the Recombined Nucleic Acid

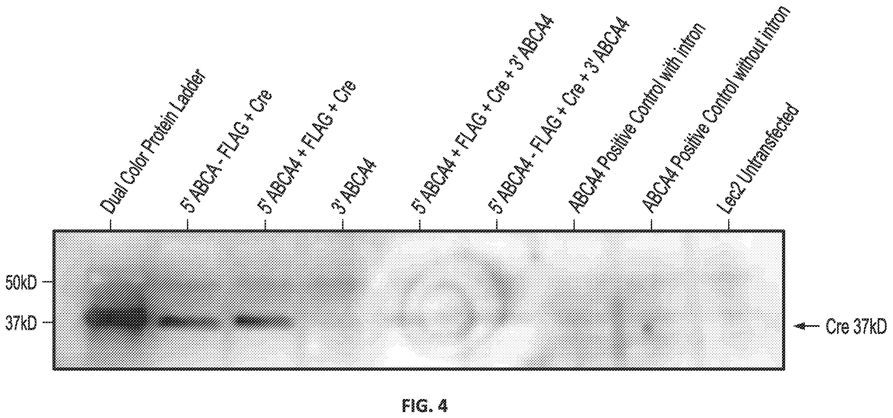

The expression level of Cre recombinase in cells containing the plasmids was analyzed **(****FIG. 4****).** Lec2 cells were transfected with various plasmids and controls as described in Example 2, and 48 hours after transfection, cell samples were processed using sample buffer containing 4M urea, and stable expression of Cre recombinase was quantified by Western blotting using 3% milk as blocking agent, rabbit anti-Cre mAb (Cell signaling technology #15036) at 1:10,000 dilution as primary antibody, and goat anti-rabbit-HRP (azure) at 1:10,000 dilution as secondary antibody. Auto-cleaved Cre recombinase has a molecular weight of ~37 kD. As expected, transfection of either "5'ABCA4 -FLAG +Cre" or "5'ABCA4 +FLAG +Cre" plasmid resulted in clear expression of Cre recombinase in Lec2 cells. Remarkably, there was no detectable expression by western blot of auto-cleaved Cre recombinase in cells co-transfected with the second plasmid "3'ABCA4 +FLAG", suggesting that the dual vector system allows efficient recombination between the first and second plasmids, which in turn leads to generation of a recombinant nucleic acid containing the Cre recombinase ORF without a promoter, and therefore the Cre recombinase can no longer be expressed.

### EXAMPLE 4:

### Cre-mediated Recombination of the Bipartite Vector System Achieves Expression of Full-length ABCA4 mRNA

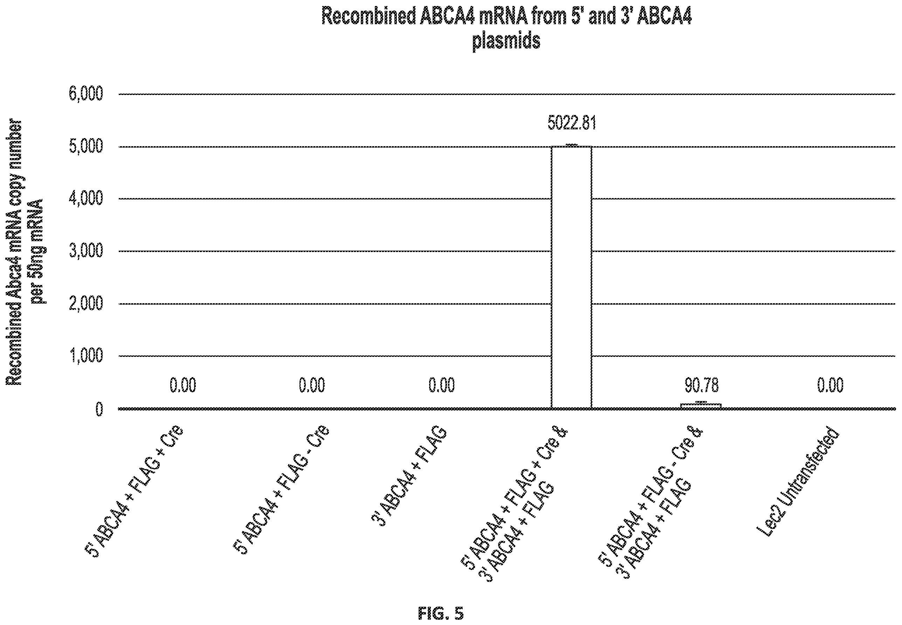

We quantified the expression levels of full-length ABCA4 mRNA in cells transfected with various plasmids. As shown in **FIG. 5****,** cells transfected with a dual vector system encoding Cre recombinase ("5'ABCA4 +FLAG +Cre" and "3'ABCA4 +FLAG") showed about 55 times higher expression level of full-length ABCA4 mRNA compared to cells transfected with a corresponding dual vector system not encoding Cre recombinase ("5'ABCA4 +FLAG -Cre" and "3'ABCA4 +FLAG"). These results show that Cre recombinase effectively mediates the recombination between the two starting vectors shortly after they are transfected into the cells and drives expression of the full-length mRNA.

### EXAMPLE 5:

### Delivery of Bipartite Vector System via Dual AAV Viral Particles Results in Expression of Full-length ABCA4 mRNA

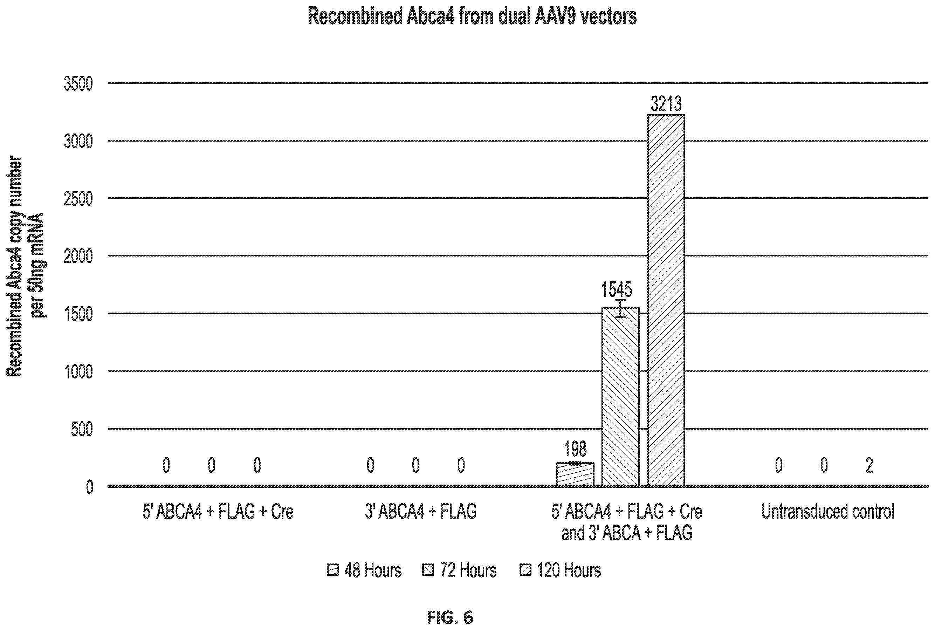

We tested whether the bipartite vector system could be delivered by AAV viral particles to express full-length ABCA4 mRNA within the cells. Two types of AAV9 viral particles were prepared using the expression cassettes described in Example 1. One type of AAV9 viral particles comprise an AAV vector genome encoding "5'ABCA4 +FLAG +Cre", and the other type of AAV9 viral particles comprise an AAV vector genome encoding "3'ABCA4 +FLAG". As shown in **FIG. 6****,** ABCA4 negative cells were transduced with the indicated AAV9 viral particles, and the expression level of the full-length ABCA4 mRNA were quantified in each group. Only cells transduced with both types of AAV9 viral particles displayed high expression level of full-length ABCA4 mRNA, and the expression level continued to increase for over 120 hours post-transduction. This result demonstrated that AAV viral particles can effectively deliver the bipartite vector system in the same cell and recombine so that the mRNA payload is properly reconstituted by the system.

## Claims

1. An AAV vector comprising, in 5' to 3' orientation:
(a) a 5' AAV inverted terminal repeat;
(b) a promoter;
(c) a 5' portion of a transgene;
(d) a splice donor (SD) site;
(e) a recombination site;
(f) a polynucleotide encoding a recombinase;
(g) a poly-A site; and
(h) a 3' AAV inverted terminal repeat,
wherein expression of the recombinase is operably linked to the promoter.

2. The AAV vector of claim 1, wherein the recombinase:
(i) is a Cre recombinase; and/or
(ii) comprises a nuclear localization sequence (NLS).

3. The AAV vector of claim 2, wherein the recombination site comprises a LoxP71 sequence.

4. The AAV vector of any one of claims 1-3, wherein the AAV vector genome comprises:
(i) a polynucleotide encoding an internal cleavage polypeptide located between the recombination site and the polynucleotide encoding the recombinase, and wherein the 5' portion of the transgene, the polynucleotide encoding the internal cleavage polypeptide, and the polynucleotide encoding the recombinase are in the same reading frame;
optionally, wherein the internal cleavage polypeptide is a self-cleaving peptide selected from the group consisting of T2A, P2A, E2A and F2A; or
(ii) an internal ribosome entry site (IRES) located between the recombination site and the polynucleotide encoding the recombinase, and wherein the IRES is operably linked to the polynucleotide encoding the recombinase.

5. The AAV vector of any one of claims 1-4, wherein the transgene encodes a polypeptide.

6. The AAV vector of claim 5, wherein the polypeptide is an ABCA4 protein.

7. The AAV vector of any one of claims 1-6, wherein the promoter is
(i) a Rous sarcoma virus (RSV) LTR promoter (optionally with the RSV enhancer), a cytomegalovirus (CMV) promoter, an SV40 promoter, a dihydrofolate reductase promoter, a beta-actin promoter, a phosphoglycerol kinase (PGK) promoter, a U6 promoter, an H1 promoter, a CAG promoter, a hybrid chicken beta-actin promoter, an MeCP2 promoter, an EF1 promoter, a ubiquitous chicken β-actin hybrid (CBh) promoter, a U1a promoter, a U1b promoter, an MeCP2 promoter, an MeP418 promoter, an MeP426 promoter, a minimal MeCP2 promoter, a VMD2 promoter, an mRho promoter, a EFla promoter, a Ubc promoter, a human β-actin promoter, a TRE promoter, an Ac5 promoter, a Polyhedrin promoter, a CaMKIIa promoter, Gall promoter, a TEF1 promoter, a GDS promoter, an ADH1 promoter, a Ubi promoter, or an α-1-antitrypsin (hAAT) promoter; or
(ii) a human rhodopsin kinase (RK) promoter, a human interphotoreceptor binding protein promoter (IRBP), a human red/green opsin promoter (pR2.1), a human blue opsin promoter (HB), a mouse opsin promoter (mOP), a mouse short wavelength opsin promoter (mBP), or a human rod cGMP phosphodiesterase β-subunit promoter (βPDE).

8. An AAV viral particle, comprising
(i) an AAV capsid, wherein the AAV capsid comprises an AAV capsid protein; and
(ii) the AAV vector of any one of claims 1-7;
optionally, wherein the AAV capsid protein is at least 70%, 80%, 90%, 99% or 100% identical to a sequence selected from SEQ ID NOs: 1-3, 67, 71, 196, 205 and 206.

9. A pharmaceutical composition comprising the AAV vector of any one of claims 1-7 or the AAV viral particle of claim 8.

10. A pharmaceutical composition comprising
(i) a first AAV viral particle comprising the AAV vector of any one of claims 1-7; and
(ii) a second AAV viral particle comprising a second AAV vector comprising, in 5' to 3' orientation:
(a) a 5' AAV inverted terminal repeat;
(b) a recombination site;
(c) a splice acceptor (SA) site;
(d) a 3' portion of the transgene;
(e) a poly-A site; and
(f) a 3' AAV inverted terminal repeat;
wherein the recombination site in the AAV vector of the first AAV viral particle and the recombination site in the second AAV vector of the second AAV viral particle prevent backwards recombination.

11. A first AAV viral particle for use, in combination with a second AAV viral particle, in a method of treating a subject having a disease or disorder caused by a gene defect, wherein:
(1) the first AAV viral particle comprises:
(i) an AAV capsid, wherein the AAV capsid comprises a first AAV capsid protein; and
(ii) the AAV vector of any one of claims 1-7; and
(2) the second AAV viral particle comprises:
(i) an AAV capsid, wherein the AAV capsid comprises a second AAV capsid protein; and
(ii) a second AAV vector wherein the second AAV vector comprises, in 5' to 3' orientation:
(a) a 5' AAV inverted terminal repeat;
(b) a recombination site;
(c) a splice acceptor (SA) site;
(d) a 3' portion of the transgene;
(e) a poly-A site; and
(f) a 3' AAV inverted terminal repeat.

12. The first AAV viral particle for use of claim 11, wherein administration of the first AAV viral particle and the second AAV viral particle leads to recombination of the first AAV vector and the second AAV vector via the recombination site in the AAV vector of the first AAV viral particle and the recombination site in the second AAV vector of the second AAV viral particle;
optionally, wherein administration of the first AAV viral particle and the second AAV viral particle leads to expression of the polypeptide;
optionally, wherein the first AAV viral particle and the second AAV viral particle are co-administered or sequentially administered;
optionally, wherein the viral particles are administered by subretinal injection.

13. The first AAV viral particle for use of any one of claims 11-12, wherein the gene defect is an ABCA4 gene defect;
optionally, wherein the ABCA4 gene defect results in one or more conditions selected from the group consisting of reduced expression of ABCA4 protein, eliminated expression of ABCA4 protein, expression of a mutated ABCA4 protein, and reduced function of ABCA4 protein.

14. An *ex vivo* method of expressing a polypeptide in a cell, comprising:
(1) transducing the cell with a first AAV viral particle comprising the AAV vector of any one of claims 1-7, wherein the transgene encodes the polypeptide; and
(2) transducing the cell with a second AAV viral particle comprising a second AAV vector wherein the second AAV vector genome comprises, in 5' to 3' orientation
(a) a 5' AAV inverted terminal repeat;
(b) a recombination site;
(c) a splice acceptor (SA) site;
(d) a 3' portion of the transgene;
(e) a poly-A site; and
(f) a 3' AAV inverted terminal repeat;
optionally; wherein no stable expression of the recombinase is detected in the transduced cell; optionally, wherein the polypeptide is an ABCA4 protein.

15. A transduced cell obtained by the ex vivo method of claim 14, comprising:
(i) a defective genomic copy of a gene;
(ii) a first recombinant nucleic acid comprising, in 5' to 3' orientation:
(a) a 5' AAV inverted terminal repeat;
(b) a promoter;
(c) a 5' portion of a transgene;
(d) a splice donor (SD) site;
(e) a recombination site;
(f) a splice acceptor (SA) site;
(g) a 3' portion of the transgene;
(h) a poly-A site; and
(i) a 3' AAV inverted terminal repeat;
wherein the transgene has a length of 4.6-7.7 kb; and
(iii) a second recombinant nucleic acid comprising, in 5' to 3' orientation: a 5' AAV inverted terminal repeat, a polynucleotide encoding a recombinase, and a 3' AAV inverted terminal repeat; wherein the second recombinant nucleic acid lacks a promoter;
optionally, wherein no stable expression of the recombinase is detected in the transduced cell; optionally, wherein the recombinase is a Cre recombinase; optionally, wherein the transgene encodes an ABCA4 gene; optionally, wherein the cell is an *ex vivo* cell.

## Patentansprüche

1. AAV-Vektor, umfassend in 5'-nach-3'-Orientierung:
(a) ein 5'-AAV-Inverted-Terminal-Repeat;
(b) einen Promotor;
(c) einen 5'-Teil eines Transgens;
(d) eine Spleiß-Donor(SD)-Stelle;
(e) eine Rekombinationsstelle;
(f) ein Polynukleotid, das eine Rekombinase codiert;
(g) eine Poly-A-Stelle; und
(h) ein 3'-AAV-Inverted-Terminal-Repeat;
wobei die Expression der Rekombinase funktionell mit dem Promotor verknüpft ist.

2. AAV-Vektor nach Anspruch 1, wobei die Rekombinase:
(i) eine Cre-Rekombinase ist; und/oder
(ii) eine Kernlokalisierungssequenz (NLS= nuclear localization sequence) umfasst.

3. AAV-Vektor nach Anspruch 2, wobei die Rekombinationsstelle eine LoxP71-Sequenz umfasst.

4. AAV-Vektor nach einem der Ansprüche 1-3, wobei das AAV-Vektorgenom Folgendes umfasst:
(i) ein Polynukleotid, das ein internes Spaltpolypeptid codiert, das sich zwischen der Rekombinationsstelle und dem Polynukleotid befindet, das für die Rekombinase codiert, und wobei der 5'-Teil des Transgens, das Polynukleotid, das das interne Spaltpolypeptid codiert, und das Polynukleotid, das die Rekombinase codiert, in demselben Leseraster liegen;
gegebenenfalls wobei es sich bei dem internen Spaltpolypeptid um ein selbstspaltendes Peptid handelt, das aus der Gruppe bestehend aus T2A, P2A, E2A und F2A ausgewählt ist; oder
(ii) eine interne Ribosomeneintrittsstelle (IRES), die sich zwischen der Rekombinationsstelle und dem Polynukleotid befindet, das die Rekombinase codiert, und wobei die IRES mit dem Polynukleotid, das die Rekombinase codiert, funktionell verknüpft ist.

5. AAV-Vektor nach einem der Ansprüche 1-4, wobei das Transgen ein Polypeptid codiert.

6. AAV-Vektor nach Anspruch 5, wobei es sich bei dem Polypeptid um ein ABCA4-Protein handelt.

7. AAV-Vektor nach einem der Ansprüche 1-6, wobei es sich bei dem Promotor um Folgendes handelt:
(i) einen Rous-Sarkom-Virus(RSV)-LTR-Promotor (gegebenenfalls mit dem RSV-Enhancer), einen Cytomegalovirus(CMV)-Promotor, einen SV40-Promotor, einen Dihydrofolatreduktase-Promotor, einen Beta-Aktin-Promotor, einen Phosphoglycerinkinase(PGK)-Promotor, einen U6-Promotor, einen H1-Promotor, einen CAG-Promotor, einen Hybrid-Hühner-beta-Aktin-Promotor, einen MeCP2-Promotor, einen EF1-Promotor, einen ubiquitären Hühner-β-Aktin-Hybrid(CBh)-Promotor, einen U1a-Promotor, einen U1b-Promotor, einen MeCP2-Promotor, einen MeP418-Promotor, einen MeP426-Promotor, einen minimalen MeCP2-Promotor, einen VMD2-Promotor, einen mRho-Promotor, einen EF1a-Promotor, einen Ubc-Promotor, einen menschlichen β-Aktin-Promotor, einen TRE-Promotor, einen Ac5-Promotor, einen Polyhedrin-Promotor, einen CaMKIIa-Promotor, Gall-Promotor, einen TEF1-Promotor, einen GDS-Promotor, einen ADH1-Promotor, einen Ubi-Promotor oder einen α-1-Antitrypsin(hAAT)-Promotor; oder
(ii) einen menschlichen Rhodopsinkinase(RK)-Promotor, einen menschlichen Interphotorezeptor-Bindungsproteinpromotor (IRBP), einen menschlichen Rot-/Grün-Opsin-Promotor (pR2.1), einen menschlichen Blau-Opsin-Promotor (HB), einen Maus-Opsin-Promotor (mOP), einen kurzwelligen Maus-Opsin-Promotor (mBP) oder einen menschlichen Stäbchen-cGMP-Phosphodiesterase-β-Untereinheitspromotor (βPDE).

8. AAV-Viruspartikel, umfassend
(i) ein AAV-Kapsid, wobei das AAV-Kapsid ein AAV-Kapsidprotein umfasst; und
(ii) den AAV-Vektor nach einem der Ansprüche 1-7; gegebenenfalls wobei das AAV-Kapsidprotein mindestens 70 %, 80 %, 90 %, 99 % oder 100 % identisch zu einer Sequenz ist, die aus SEQ ID NOS: 1-3, 67, 71, 196, 205 und 206 ausgewählt ist.

9. Pharmazeutische Zusammensetzung, umfassend den AAV-Vektor nach einem der Ansprüche 1-7 oder das AAV-Viruspartikel nach Anspruch 8.

10. Pharmazeutische Zusammensetzung, umfassend
(i) ein erstes AAV-Viruspartikel, das den AAV-Vektor nach einem der Ansprüche 1-7 umfasst; und
(ii) ein zweites AAV-Viruspartikel, das einen zweiten AAV-Vektor umfasst, der in 5'-nach-3'-Orientierung Folgendes umfasst:
(a) ein 5'-AAV-Inverted-Terminal-Repeat;
(b) eine Rekombinationsstelle;
(c) eine Spleiß-Acceptor(SA)-Stelle;
(d) einen 3'-Teil des Transgens;
(e) eine Poly-A-Stelle; und
(f) ein 3'-AAV-Inverted-Terminal-Repeat;
wobei die Rekombinationsstelle im AAV-Vektor des ersten AAV-Viruspartikels und die Rekombinationsstelle im zweiten AAV-Vektor des zweiten AAV-Viruspartikels eine Rückwärts-Rekombination verhindern.

11. Erstes AAV-Viruspartikel zur Verwendung in Kombination mit einem zweiten AAV-Viruspartikel bei einem Verfahren zum Behandeln eines Individuums mit einer Krankheit oder Störung, die durch einen Gendefekt verursacht wird, wobei:
(1) das erste AAV-Viruspartikel Folgendes umfasst:
(i) ein AAV-Kapsid, wobei das AAV-Kapsid ein erstes AAV-Kapsidprotein umfasst; und
(ii) den AAV-Vektor nach einem der Ansprüche 1-7; und
(2) das zweite AAV-Viruspartikel Folgendes umfasst:
(i) ein AAV-Kapsid, wobei das AAV-Kapsid ein zweites AAV-Kapsidprotein umfasst; und
(ii) einen zweiten AAV-Vektor, wobei der zweite AAV-Vektor in 5'-nach-3'-Orientierung Folgendes umfasst:
(a) ein 5'-AAV-Inverted-Terminal-Repeat;
(b) eine Rekombinationsstelle;
(c) eine Spleiß-Acceptor(SA)-Stelle;
(d) einen 3'-Teil des Transgens;
(e) eine Poly-A-Stelle; und
(f) ein 3'-AAV-Inverted-Terminal-Repeat.

12. Erstes AAV-Viruspartikel zur Verwendung nach Anspruch 11, wobei die Verabreichung des ersten AAV-Viruspartikels und des zweiten AAV-Viruspartikels zur Rekombination des ersten AAV-Vektors und des zweiten AAV-Vektors über die Rekombinationsstelle im AAV-Vektor des ersten AAV-Viruspartikels und die Rekombinationsstelle im zweiten AAV-Vektor des zweiten AAV-Viruspartikels führt;
gegebenenfalls wobei die Verabreichung des ersten AAV-Viruspartikels und des zweiten AAV-Viruspartikels zur Expression des Polypeptids führt;
gegebenenfalls wobei das erste AAV-Viruspartikel und das zweite AAV-Viruspartikel gemeinsam oder nacheinander verabreicht werden;
gegebenenfalls wobei die Viruspartikel mittels subretinaler Injektion verabreicht werden.

13. Erstes AAV-Viruspartikel zur Verwendung nach einem der Ansprüche 11-12, wobei es sich bei dem Gendefekt um einen ABCA4-Gendefekt handelt;
gegebenenfalls wobei der ABCA4-Gendefekt zu einem oder mehreren Leiden führt, die aus der Gruppe bestehend aus reduzierter ABCA4-Proteinexpression, beseitigter ABCA4-Proteinexpression, Expression eines mutierten ABCA4-Proteins und reduzierter Funktion des ABCA4-Proteins ausgewählt sind.

14. Ex-vivo-Verfahren zum Exprimieren eines Polypeptids in einer Zelle, umfassend:
(1) Transduzieren der Zelle mit einem ersten AAV-Viruspartikel, das den AAV-Vektor nach einem der Ansprüche 1-7 umfasst, wobei das Transgen das Polypeptid codiert; und
(2) Transduzieren der Zelle mit einem zweiten AAV-Viruspartikel, das einen zweiten AAV-Vektor umfasst, wobei das zweite AAV-Vektorgenom in 5'-nach-3'-Orientierung Folgendes umfasst:
(a) ein 5'-AAV-Inverted-Terminal-Repeat;
(b) eine Rekombinationsstelle;
(c) eine Spleiß-Acceptor(SA)-Stelle;
(d) einen 3'-Teil des Transgens;
(e) eine Poly-A-Stelle; und
(f) ein 3'-AAV-Inverted-Terminal-Repeat;
gegebenenfalls wobei keine stabile Expression der Rekombinase in der transduzierten Zelle nachgewiesen wird; gegebenenfalls wobei es sich bei dem Polypeptid um ein ABCA4-Protein handelt.

15. Transduzierte Zelle, erhalten durch das Ex-vivo-Verfahren nach Anspruch 14, umfassend:
(i) eine defekte genomische Kopie eines Gens;
(ii) eine erste rekombinante Nukleinsäure, die in 5'-nach-3'-Orientierung Folgendes umfasst:
(a) ein 5'-AAV-Inverted-Terminal-Repeat;
(b) einen Promotor;
(c) einen 5'-Teil eines Transgens;
(d) eine Spleiß-Donor(SD)-Stelle;
(e) eine Rekombinationsstelle;
(f) eine Spleiß-Acceptor(SA)-Stelle;
(g) einen 3'-Teil des Transgens;
(h) eine Poly-A-Stelle; und
(i) ein 3'-AAV-Inverted-Terminal-Repeat;
wobei das Transgen eine Länge von 4,6-7,7 kb aufweist; und
(iii) eine zweite rekombinante Nukleinsäure, die in 5'-nach-3'-Orientierung Folgendes umfasst: ein 5'-AAV-Inverted-Terminal-Repeat, ein Polynukleotid, das eine Rekombinase codiert, und ein 3'-AAV-Inverted-Terminal-Repeat; wobei der zweiten rekombinanten Nukleinsäure ein Promotor fehlt;
gegebenenfalls wobei keine stabile Expression der Rekombinase in der transduzierten Zelle nachgewiesen wird; gegebenenfalls wobei es sich bei der Rekombinase um eine Cre-Rekombinase handelt; gegebenenfalls wobei das Transgen ein ABCA4-Gen codiert; gegebenenfalls wobei es sich bei der Zelle um eine Ex-vivo-Zelle handelt.

## Revendications

1. Vecteur AAV comprenant, dans une orientation 5'-3' :
(a) une répétition terminale inversée AAV 5' ;
(b) un promoteur ;
(c) une partie 5' d'un transgène ;
(d) un site donneur d'épissure (SD) ;
(e) un site de recombinaison ;
(f) un polynucléotide codant pour une recombinase ;
(g) un site poly-A ; et
(h) une répétition terminale inversée AAV 3' ;
dans lequel l'expression de la recombinase est liée fonctionnellement au promoteur.

2. Vecteur AAV selon la revendication 1, dans lequel la recombinase :
(i) est une recombinase CRE ; et/ou
(ii) comprend une séquence de localisation nucléaire (NLS).

3. Vecteur AAV selon la revendication 2, dans lequel le site de recombinaison comprend une séquence LoxP71.

4. Vecteur AAV selon l'une quelconque des revendications 1 à 3, le génome du vecteur AAV comprenant :
(i) un polynucléotide codant pour un polypeptide de clivage interne situé entre le site de recombinaison et le polynucléotide codant pour la recombinase, et la partie 5' du transgène, le polynucléotide codant pour le polypeptide de clivage interne et le polynucléotide codant pour la recombinase étant dans le même cadre de lecture ;
facultativement, dans lequel le polypeptide de clivage interne est un peptide autoclivant choisi dans le groupe constitué par T2A, P2A, E2A et F2A ; ou
(ii) un site d'entrée de ribosome interne (IRES) situé entre le site de recombinaison et le polynucléotide codant pour la recombinase, et l'IRES étant lié fonctionnellement au polynucléotide codant pour la recombinase.

5. Vecteur AAV selon l'une quelconque des revendications 1 à 4, dans lequel le transgène code pour un polypeptide.

6. Vecteur AAV selon la revendication 5, dans lequel le polypeptide est une protéine ABCA4.

7. Vecteur AAV selon l'une quelconque des revendications 1 à 6, dans lequel le promoteur est
(i) un promoteur LTR du virus du sarcome de Rous (RSV) (facultativement avec l'amplificateur de RSV), un promoteur de cytomégalovirus (CMV), un promoteur SV40, un promoteur de dihydrofolate réductase, un promoteur de bêta-actine, un promoteur de phosphoglycérol kinase (PGK), un promoteur U6, un promoteur H1, un promoteur CAG, un promoteur hybride de la bêta-actine de poulet, un promoteur MeCP2, un promoteur EF1, un promoteur hybride de la bêta-actine de poulet ubiquiste (CBh), un promoteur U1a, un promoteur U1b, un promoteur MeCP2, un promoteur MeP418, un promoteur MeP426, un promoteur minimal MeCP2, un promoteur VMD2, un promoteur mRho, un promoteur EF1a, un promoteur UBc, un promoteur de la β-actine humaine, un promoteur TRE, un promoteur Ac5, un promoteur de la polyhédrine, un promoteur CaMKIIa, un promoteur Gal1, un promoteur TEF1, un promoteur GDS, un promoteur ADH1, un promoteur Ubi ou un promoteur de la α-1-antitrypsine (hAAT) ; ou
(ii) un promoteur de la rhodopsine kinase (RK) humaine, un promoteur de la protéine de liaison interphotorécepteur (IRBP) humaine, un promoteur de l'opsine rouge/verte humaine (pR2.1), un promoteur de l'opsine bleue humaine (HB), un promoteur de l'opsine de souris (MOP), un promoteur de l'opsine de courte longueur d'onde de souris (mBP) ou un promoteur de la sous-unité β de la phosphodiestérase du cGMP des bâtonnets humains (βPDE).

8. Particule virale AAV, comprenant
(i) une capside d'AAV, la capside d'AAV comprenant une protéine de capside d'AAV ; et
(ii) le vecteur AAV selon l'une quelconque des revendications 1 à 7 ;
Facultativement, dans laquelle la protéine de capside AAV présente au moins 70 %, 80 %, 90 %, 99 % ou 100 % d'identité avec une séquence choisie parmi SEQ ID NO : 1 à 3, 67, 71, 196, 205 et 206.

9. Composition pharmaceutique comprenant le vecteur AAV selon l'une quelconque des revendications 1 à 7 ou la particule virale AAV selon la revendication 8.

10. Composition pharmaceutique comprenant
(i) une première particule virale AAV comprenant le vecteur AAV selon l'une quelconque des revendications 1 à 7 ; et
(ii) une deuxième particule virale AAV comprenant un deuxième vecteur AAV comprenant, dans l'orientation 5'-3' :
(a) une répétition terminale inversée AAV 5' ;
(b) un site de recombinaison ;
(c) un site accepteur d'épissure (SA) ;
(d) une partie 3' du transgène ;
(e) un site poly-A ; et
(f) une répétition terminale inversée AAV 3' ;
dans laquelle le site de recombinaison dans le vecteur AAV de la première particule virale AAV et le site de recombinaison dans le deuxième vecteur AAV de la deuxième particule virale AAV empêchent une recombinaison inverse.

11. Première particule virale AAV pour une utilisation, en combinaison avec une deuxième particule virale AAV, dans une méthode de traitement d'un sujet souffrant d'une maladie ou d'un trouble causé par un défaut génétique, **caractérisée en ce que** :
(1) la première particule virale AAV comprend :
(i) une capside d'AAV, la capside d'AAV comprenant une première protéine de capside d'AAV ; et
(ii) le vecteur AAV selon l'une quelconque des revendications 1 à 7 ; et
(2) la deuxième particule virale AAV comprend :
(i) une capside d'AAV, la capside d'AAV comprenant une deuxième protéine de capside d'AAV ; et
(ii) un deuxième vecteur AAV, le deuxième vecteur AAV comprenant, dans l'orientation 5'-3' :
(a) une répétition terminale inversée AAV 5' ;
(b) un site de recombinaison ;
(c) un site accepteur d'épissure (SA) ;
(d) une partie 3' du transgène ;
(e) un site poly-A ; et
(f) une répétition terminale inversée AAV 3'.

12. Première particule virale AAV selon la revendication 11, dans laquelle l'administration de la première particule virale AAV et de la deuxième particule virale AAV conduisant à la recombinaison du premier vecteur AAV et du deuxième vecteur AAV via le site de recombinaison dans le vecteur AAV de la première particule virale AAV et le site de recombinaison dans le deuxième vecteur AAV de la deuxième particule virale AAV ;
facultativement, l'administration de la première particule virale AAV et de la deuxième particule virale AAV conduisant à l'expression du polypeptide ;
facultativement, la première particule virale AAV et la deuxième particule virale AAV étant coadministrées ou administrées séquentiellement ;
facultativement, les particules virales sont administrées par injection sous-rétinienne.

13. Première particule virale AAV selon l'une quelconque des revendications 11 à 12, dans laquelle le défaut génétique est un défaut du gène ABCA4 ;
facultativement, le défaut du gène ABCA4 conduisant à une ou plusieurs conditions choisies dans le groupe constitué par une expression réduite de la protéine ABCA4, une expression supprimée de la protéine ABCA4, une expression d'une protéine ABCA4 mutée et une fonction réduite de la protéine ABCA4.

14. Procédé *ex vivo* d'expression d'un polypeptide dans une cellule, comprenant :
(1) la transduction de la cellule avec une première particule virale AAV comprenant le vecteur AAV selon l'une quelconque des revendications 1 à 7, le transgène codant pour le polypeptide ; et
(2) la transduction de la cellule avec une deuxième particule virale AAV comprenant un deuxième vecteur AAV, le deuxième génome du vecteur AAV comprenant, dans l'orientation 5'-3'
(a) une répétition terminale inversée AAV 5' ;
(b) un site de recombinaison ;
(c) un site accepteur d'épissure (SA) ;
(d) une partie 3' du transgène ;
(e) un site poly-A ; et
(f) une répétition terminale inversée AAV 3' ;
facultativement, aucune expression stable de la recombinase n'étant détectée dans la cellule transduite ; facultativement, le polypeptide étant une protéine ABCA4.

15. Cellule transduite obtenue par le procédé ex vivo selon la revendication 14, comprenant :
(i) une copie génomique défectueuse d'un gène ;
(ii) un premier acide nucléique recombinant comprenant, dans l'orientation 5'-3' :
(a) une répétition terminale inversée AAV 5' ;
(b) un promoteur ;
(c) une partie 5' d'un transgène ;
(d) un site donneur d'épissure (SD) ;
(e) un site de recombinaison ;
(f) un site accepteur d'épissure (SA) ;
(g) une partie 3' du transgène ;
(h) un site poly-A ; et
(i) une répétition terminale inversée AAV 3' ;
dans laquelle le transgène a une longueur de 4,6 à 7,7 kb ; et
(iii) un deuxième acide nucléique recombinant comprenant, dans l'orientation 5'-3' : une répétition terminale inversée AAV 5', un polynucléotide codant pour une recombinase et une répétition terminale inversée AAV 3' ; le deuxième acide nucléique recombinant étant dépourvu de promoteur ;
facultativement, aucune expression stable de la recombinase n'étant détectée dans la cellule transduite ; facultativement, la recombinase étant une recombinase Cre ; facultativement, le transgène codant pour un gène ABCA4 ; facultativement, la cellule étant une cellule ex *vivo.*
